# EUROPEAN PATENT SPECIFICATION

(11) **EP 4 507 614 B1**
(45) Date of publication and mention of the grant of the patent: **08.07.2026**
(21) Application number: 23720193.4
(22) Date of filing: 22.03.2023
(51) Int. Cl.: A61B 34/30, A61F 2/40, A61B 34/00, A61B 34/10

(54) **PATIENT-SPECIFIC ORTHOPEDIC IMPLANT EVALUATION**
PATIENTENSPEZIFISCHE BEURTEILUNG EINES ORTHOPÄDISCHEN IMPLANTATS
ÉVALUATION D'IMPLANT ORTHOPÉDIQUE SPÉCIFIQUE À UN PATIENT

(30) Priority: 12.04.2022 US 202263330095 P
(43) Date of publication of application: 19.02.2025
(73) Proprietor: Zimmer, Inc., Warsaw, Indiana 46580 (US)
(72) Inventor: FAVRE, Philippe, 8047 Zurich (CH); BISCHOFF, Jeffrey E., Holland, Michigan 49424 (US); HENDERSON, Adam D., 8400 Winterthur (CH); MUERI, Christine Mirja, 8405 Winterthur (CH); MAQUER, Ghislain, 8050 Zurich (CH); DHARIA, Mehul, Fayetteville, Arkansas 72703 (US); SUMMERS, Rodney, Ann Arbor, Michigan 48108 (US)
(74) Representative: Taor, Simon Edward William
(86) International application number: PCT/US2023/015930
(87) International publication number: WO 2023/200562

(56) References cited:
- WO-A1-2018/067966
- WO-A1-2020/123709
- YI-KING CHOI ET AL: "VISBONE: 3D visualization of bone mineral density", COMPUTER GRAPHICS AND APPLICATIONS, 1999. PROCEEDINGS. SEVENTH PACIFIC CONFERENCE ON SEOUL, SOUTH KOREA 5-7 OCT. 1999, LOS ALAMITOS, CA, USA,IEEE COMPUT. SOC, US, 5 October 1999 (1999-10-05), pages 138 - 146, 321, XP010359458, ISBN: 978-0-7695-0293-9, DOI: 10.1109/PCCGA.1999.803357

## Description

### CLAIM OF PRIORITY

This application claims the benefit of U.S. Provisional Patent Application Serial No. 63/330,095, filed on April 12, 2022, the benefit of priority of which is claimed hereby.

### TECHNICAL FIELD

The present disclosure is directed to systems, devices and methods for use in planning and performing arthroplasty procedures, such as those using implants to be affixed in bone. More particularly, the present disclosure is directed to using patient-specific anatomical information to improve prosthetic component fit and performance, such as by using bone density information.

### BACKGROUND

Imaging of anatomical features can be useful in preparing for and performing surgical procedures. For example, patient-specific instruments can be derived from patient imaging and robotic surgical systems can be configured to track anatomy of a patient based on registration with patient imaging.

Patient-specific instruments have been successfully deployed for many surgical procedures. By creating three-dimensional (3D) models of anatomy of a patient from medical images, surgeries can be customized using virtual 3D surgical planning for specific patients. The virtual 3D surgical planning can be used to produce patient-specific prosthetics, implants, cutting guides and instruments. Typically, patient-specific devices include patient-specific surfaces that fit over the anatomy of the specific patient in a unique way to reduce misalignment and allow for precise replication of the planned surgery as compared to arthroplasty with conventional or standard instrumentation.

In robotic surgical systems, the shape of the anatomy in the patient imaging can be registered with another frame of reference, such as the physical space of an operating room where the robotic surgical system is located. Robotic surgical arms can be used to hold various instruments in place in a desired orientation relative to both the anatomy and operating room during a procedure so that movement of an instrument in the operating room relative to the anatomy can be tracked on the anatomic imaging based on movement of the robotic surgical arm.

There is a continuing desire to improve the planning and execution of orthopedic implant procedures to improve fit and performance for the needs of specific patients.

Pat. Nos. US 10,736,539; US 10,512,451; US 10,258,256; and US 10,130,478 to Mahfouz describe various methods of planning for orthopedic implant procedures.

WO2020123709 discloses a surgical planning system for use in surgical procedures to repair an anatomy of interest includes a preplanning system to generate a virtual surgical plan and a mixed reality system that includes a visualization device wearable by a user to view the virtual surgical plan projected in a real environment. The virtual surgical plan includes a 3D virtual model of the anatomy of interest. When wearing the visualization device, the user can align the 3D virtual model with the real anatomy of interest, thereby achieving a registration between details of the virtual surgical plan and the real anatomy of interest.

WO2018067966 discloses methods, systems and devices for pre-operatively planned total or partial joint surgery including, for example, anatomic and reverse shoulder surgery guides and implants.

YI-KING CHOI ET AL: "VISBONE: 3D visualization of bone mineral density", COMPUTER GRAPHICS AND APPLICATIONS, 1999. PROCEEDINGS. SEVENTH PACIFIC CONFERENCE ON SEOUL, SOUTH KOREA 5-7 OCT. 1999, LOS ALAMITOS, CA, USA,IEEE COMPUT. SOC, US, 5 October 1999 (1999-10-05), pages 138-146, 321, XP010359458, discloses a system for 3D visualization of bone mineral density.

### OVERVIEW

The present inventors have recognized that, in surgical procedures planned using patient imaging, there is a desire to match the actual anatomy of the patient more closely, including soft tissue, to planning models derived from the patient imaging. Furthermore, the present inventors have recognized that there is a need to reduce the occurrence of adverse outcomes during implantation of the prosthetic component and subsequently during use of the prosthetic implant by the patient.

The present inventors have recognized, among other things, that problems to be solved with traditional arthroplasty procedures involve evaluating properties of the bone tissue, such as density, pre-operatively and intra-operatively in order to improve orthopedic implant selection, bone resection placement and orthopedic implant placement, as well as post-operative outcomes. For example, problems associated with not knowing bone density or improperly assessing bone density involve 1) selecting suboptimal locations for altering the bone (e.g., locations where unhealthy or less dense bone engages the orthopedic implant), 2) damaging the bone of the patient during bone altering procedures (e.g., cutting, drilling, resecting), 3) damaging the bone during implant placement (e.g., inserting the implant with excessive impact force), and 4) adverse post-operative effects associated with insufficient boney support of the implant (e.g., loosening, stress shielding and dissociation).

The present subject matter can provide solutions to these and other problems, such as by providing solutions for allowing surgeons or surgical planners to obtain an indication of bone density pre-operatively or intra-operatively as well as predictive feedback regarding outcomes of the procedure. The solutions can include one or more of the following options: use of surgery planning systems and software to A) obtain three-dimensional bone density information; B) run pre-operative and intra-operative procedure simulations to evaluate patient-specific implant scenarios [including the selection of an implant, the location of cut planes or other types of bone preparation, and the location of the implant on the cut plane or within the bone, etc.] based on bone density information, C) run artificial-intelligence-powered models to evaluate patient-specific implant scenarios based on bone density information including previously run simulations, D) to evaluate different risk factors based on bone density information; and E) to evaluate post-operative risk factors based on bone density information and provide recommendations.

According to an aspect, there is provided a surgical system as set out in claim 1. Additional features are set out in claims 2 to 10. According to an aspect, there is provided a method as set out in claim 11. Additional features are set out in claims 12 to 15.

### BRIEF DESCRIPTION OF THE DRAWINGS

FIG. 1 is a diagrammatic view of an operating room including a robot-assisted surgical system comprising a robotic arm, a computing system and a tracking system with which the systems, devices and methods of the present disclosure can be implemented.
FIG. 2 is a partial cross-sectional view of a prosthetic humeral head implant having a stem implanted into a humeral bone.
FIG. 3A is a perspective bottom view of a stemless humeral head implant showing fixation fins.
FIG. 3B is a perspective top view of the stemless humeral head implant of FIG. 3A showing a coupler for a humeral head prosthesis.
FIG. 4A is a schematic front view of a humeral bone and a scapula bone of a shoulder joint positioned within in imaging system.
FIG. 4B is a schematic view of an x-ray image of the shoulder joint of FIG. 4A with a templating image of a stemless humeral head implant and a bone density scale.
FIG. 5 is a block diagram illustrating steps of methods for producing a patient-specific bone model that can be used in obtaining and evaluating bone density information in planning and executing procedures for fixating prostheses in bone.
FIGS. 6A - 6G illustrate various steps in methods of predicting the fixation of prostheses in bone using modeling and simulating systems of the present disclosure.
FIG. 7 is a schematic view of a display screen for a surgical system showing visual indicators of different patient risks for a variety of implant conditions.
FIG. 8 is a block diagram illustrating steps of methods for training and utilizing an artificial intelligence engine for running prosthesis fixation simulations.
FIG. 9 is a schematic view of a video display device and a mobile computing device providing output indicia comprising scaled fixation feedback.
FIG. 10A is a schematic view of a display screen including digital planning tools for predicting fixation of an implant, the digital planning tools comprising a virtual bone model and a virtual prosthesis.
FIG. 10B is a schematic view of a display screen including digital planning tools for predicting fixation of an implant, the digital planning tools comprising a patient-specific bone density model.
FIG. 11 is a schematic illustration of a surgical planning system incorporating a simulation engine and an artificial intelligence engine for evaluating bone density information and predicting risk outcomes for various prostheses implanted into bone matter corresponding to the bone density information.
FIG. 12 is a block diagram of an example machine upon which any one or more of the techniques discussed herein can be performed and with which any of the devices discussed herein can be used in accordance with some embodiments.

### DETAILED DESCRIPTION

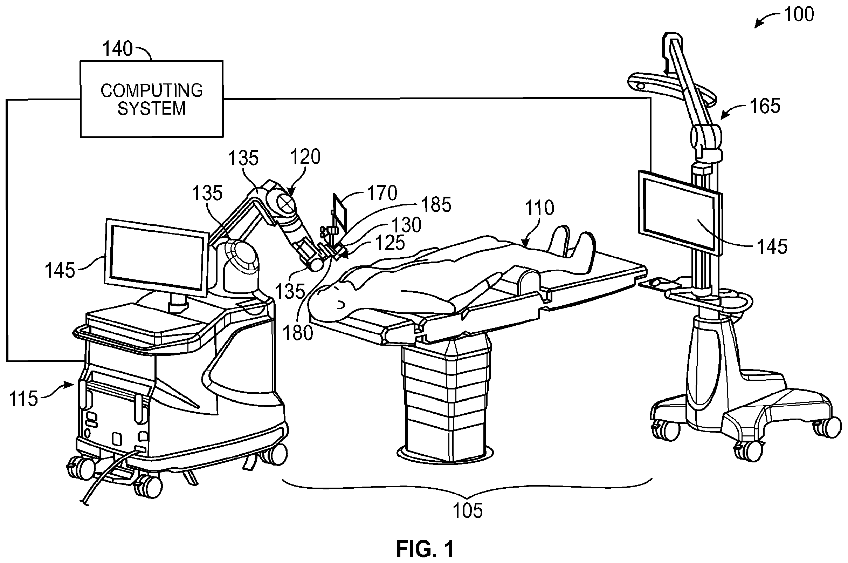

FIG. 1 illustrates surgical system 100 for operation on surgical area 105 of patient 110 in accordance with at least one example of the present disclosure. In examples, surgical area 105 can include a joint, including one or more bones. Surgical area 105 can include any surgical area of patient 110, including but not limited to the shoulder, head, elbow, hip, ankle, thumb, spine, and the like. Surgical system 100 can also include robotic system 115 with one or more robotic arms, such as robotic arm 120. As illustrated, robotic system 115 can utilize only a single robotic arm. Robotic arm 120 can be a 6 degree-of-freedom (DOF) robot arm, such as the ROSA^{®} robot from Medtech, a Zimmer Biomet Holdings, Inc. company. In some examples, robotic arm 120 can be cooperatively controlled with surgeon input on the end effector or surgical instrument, such as surgical instrument 125. In other examples, robotic arm 120 can operate autonomously. While not illustrated in FIG. 1, one or more positionable surgical support arms can be incorporated into surgical system 100 to assist in positioning and stabilizing instruments or anatomy during various procedures.

Each robotic arm 120 can rotate axially and radially and can receive a surgical instrument, or end effector, 125 at distal end 130. Surgical instrument 125 can be any surgical instrument adapted for use by the robotic system 115, including, for example, a guide tube, a holder device, a gripping device such as a pincer grip, a burring device, a reaming device, an impactor device such as a humeral head impactor, a pointer, a force-limiting device, a probe or the like. Surgical instrument 125 can be positionable by robotic arm 120, which can include multiple robotic joints, such as joints 135, that allow surgical instrument 125 to be positioned at any desired location adjacent or within a given surgical area 105. Robotic arm 120 can be used with an instrument positioning device, e.g., an instrument holder, to position an instrument in a known, desired or predetermined orientation relative to surgical area 105 based on a virtual coordinate system determined by computing system 140.

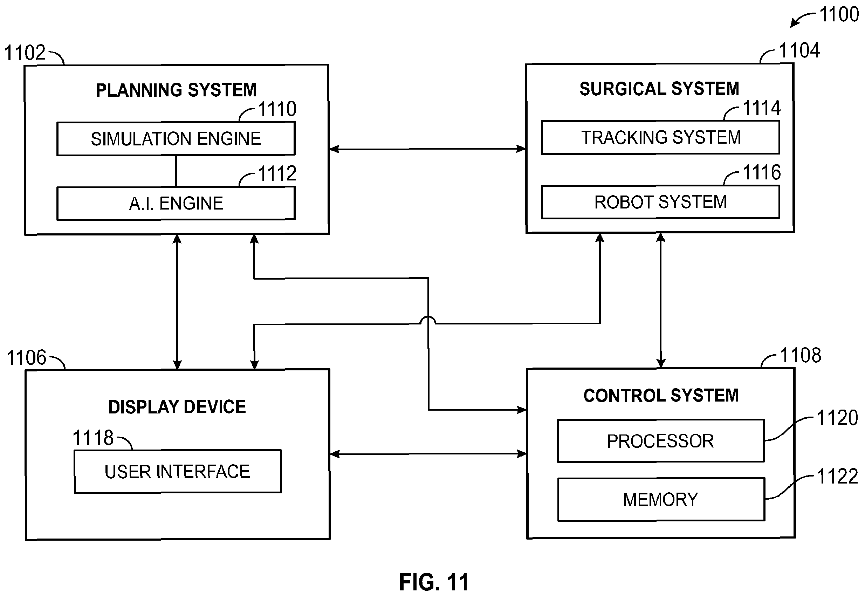

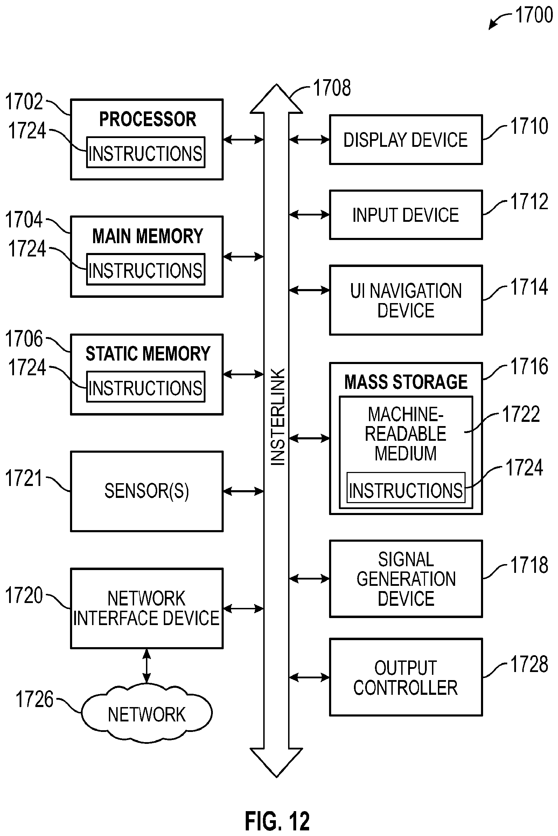

Robotic system 115 can also include computing system 140 that can operate robotic arm 120 and surgical instrument 125. Computing system 140 can include at least memory, a processing unit, and user input devices, as will be described herein, such as with reference to FIGS. 11 and 12. Computing system 140 and tracking system 165 can also include human interface devices 145 for providing images for a surgeon to be used during surgery. Computing system 140 is illustrated as a separate standalone system, but in some examples computing system 140 can be integrated into robotic system 115. Human interface devices 145 can provide images, including but not limited to three-dimensional images of bones, a glenoid, joints, prostheses, and the like, as well as bone density information for generic bones and specific bones of a patient. Human interface devices 145 can include associated input mechanisms, such as a touch screen, foot pedals, or other input devices compatible with a surgical environment.

Computing system 140 can receive pre-operative, intra-operative and post-operative medical images. These images can be received in any manner and the images can include, but are not limited to, computed tomography (CT) scans, magnetic resonance imaging (MRI), two-dimensional x-rays, three-dimensional x-rays, ultrasound, and the like. As discussed herein, these images can include, or can be modified to include, bone density information that can be used to, for example, produce three-dimensional models of anatomy of a specific patient that can indicate three-dimensional bone density. The images and three-dimensional bone density models, in examples, can be sent via a server as files attached to an email. In another example the images can be stored on an external memory device such as a memory stick and coupled to a USB port of the robotic system to be uploaded into the processing unit. In yet other examples, the images can be accessed over a network by computing system 140 from a remote storage device or service.

After receiving one or more images, computing system 140 can generate one or more virtual models related to surgical area 105, such as a three-dimensional model incorporating, for example, bone density or anatomy information. Alternatively, computing system 140 can receive virtual models of the anatomy of the patient prepared remotely. Specifically, a virtual model of the anatomy of patient 110 can be created by defining anatomical points within the image(s) and/or by fitting a statistical anatomical model to the image data. In examples, the virtual model can comprise a finite element model. Additionally, the virtual model can be created using multiple orthogonal x-ray images of the anatomy of the patient merged together to form a three-dimensional model. The virtual model, along with virtual representations of implants, can be used for calculations related to the desired height, depth, inclination angle, or version angle of an implant, stem, surgical instrument, or the like related to be utilized in surgical area 105. In another procedure type, the virtual model can be utilized to determine insertion location, trajectory, insertion force (e.g., an insertion force ceiling to avoid adversely affecting bone structure of a specific patient) and depth for inserting an instrument. The virtual model can also be used to determine bone dimensions, implant dimensions, bone fragment dimensions, bone fragment arrangements, and the like. Any model generated, including three-dimensional models, can be displayed on human interface devices 145 for reference during a surgery or used by robotic system 115 to determine motions, actions, and operations of robotic arm 120 or surgical instrument 125. Known techniques for creating virtual bone models can be utilized, such as those discussed in U.S. Patent No. 9,675,461, titled "Deformable articulating templates" or U.S. Patent No. 8,884,618, titled "Method of generating a patient-specific bone shell" both by Mahfouz, as well as other techniques known in the art.

The virtual models and three-dimensional models can include patient-specific information, such as age, gender, ethnicity, height, weight, activity level and the like. The patient-specific, three-dimensional bone models can be used to, for example, 1) determine various prostheses compatible with anatomy, e.g., soft tissue anatomy including bone density information, of the imaged patient, 2) determine locations for bone modifications in order to prepare the bone to receive a prosthetic device accounting for the density of the bone matter, 3) determine fit and fixation of a selected prosthesis with bone matter of the patient based on density of the bone matter, and 4) determine post-operative outcomes for the selected parameters of 1) - 3) based on, for example, particular activities of the imaged patient.

Computing system 140 can also communicate with tracking system 165 that can be operated by computing system 140 as a stand-alone unit. Surgical system 100 can utilize the Polaris optical tracking system from Northern Digital, Inc. of Waterloo, Ontario, Canada. Additionally, tracking system 165 can comprise the tracking system shown and described in Pub. No. US 2017/0312035, titled "Surgical System Having Assisted Navigation" to Brian M. May. Tracking system 165 can monitor a plurality of tracking elements, such as tracking elements 170, affixed to objects of interest to track locations of multiple objects within the surgical field. Tracking system 165 functions to create a virtual three-dimensional coordinate system within the surgical field for tracking patient anatomy, surgical instruments, or portions of robotic system 115. Tracking elements 170 can be tracking frames including multiple IR reflective tracking spheres, or similar optically tracked marker devices. In one example, tracking elements 170 can be placed on or adjacent one or more bones of patient 110. In other examples, tracking elements 170 can be placed on robot robotic arm 120, surgical instrument 125, and/or an implant to accurately track positions within the virtual coordinate system associated with surgical system 100. In each instance tracking elements 170 can provide position data, such as patient position, bone position, joint position, robotic arm position, implant position, or the like.

Robotic system 115 can include various additional sensors and guide devices. For example, robotic system 115 can include one or more force sensors, such as force sensor 180. Force sensor 180 can provide additional force data or information to computing system 140 of robotic system 115. Force sensor 180 can be used by a surgeon to cooperatively move robotic arm 120. For example, force sensor 180 can be used to monitor impact or implantation forces during certain operations, such as insertion of an implant stem into a intramedullary canal. Monitoring forces can assist in preventing negative outcomes through force fitting components. In other examples, force sensor 180 can provide information on soft-tissue tension in the tissues surrounding a target joint. In certain examples, robotic system 115 can also include laser pointer 185 that can generate a laser beam or array that is used for alignment of implants during surgical procedures.

In order to ensure that computing system 140 is moving robotic arm 120 in a known and fixed relationship to surgical area 105 and patient 110, the space of surgical area 105 and patient 110 can be registered to computing system 140 via a registration process involving registering fiducial markers attached to patient 110 with corresponding images of the markers in patient 110 recorded preoperatively or just prior to a surgical procedure. For example, a plurality of fiducial markers can be attached to patient 110, images of patient 110 with the fiducial markers can be taken or obtained and stored within a memory device of computing system 140. Subsequently, patient 110 with the fiducial markers can be moved into, if not already there because of the imaging, surgical area 105 and robotic arm 120 can touch each of the fiducial markers. Engagement of each of the fiducial markers can be cross-referenced with, or registered to, the location of the same fiducial marker in the images. In additional examples, patient 110 and medical images of the patient can be registered in real space using contactless methods, such as by using a laser rangefinder held by robotic arm 120 and a surface matching algorithm that can match the surface of the patient from scanning of the laser rangefinder and the surface of the patient in the medical images. As such, the real-world, three-dimensional geometry of the anatomy attached to the fiducial markers can be correlated to the anatomy in the images and movements of instruments 125 attached to robotic arm 120 based on the images will correspondingly occur in surgical area 105.

Subsequently, other instruments and devices attached to surgical system 100 can be positioned by robotic arm 120 into a known and desired orientation relative to the anatomy to implant a selected prosthesis according to the selected operative plan. The location where robotic arm 120 holds instruments and where associated resections or bone alterations are placed on a bone can be planned using patient-specific soft tissue information.

As discussed herein, surgical system 100 and in particular computing system 140 can be used to provide pre-operative or intra-operative feedback regarding fixation of implants into bone matter based on the bone density of the bone. The feedback can comprise discrete information about the bone density at specific locations in the bone matter. The feedback can also comprise information, such as risk assessments, about how a selected prosthesis can interact with the bone matter for a selected bone modification plan. The feedback can be in the form of visual indicators, such as heat maps, risk scales, color coding and the like to inform a surgical team about specific risks for a specific patient. Furthermore, the feedback can comprise discrete recommendations, such as no-go or go recommendations for specifically planned implant scenarios.

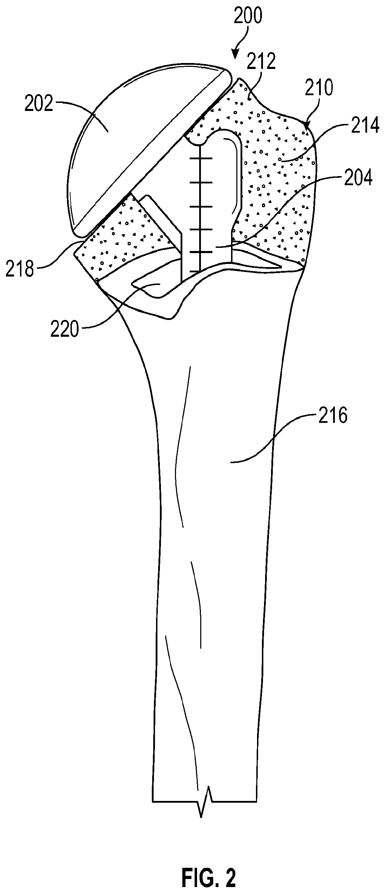

FIG. 2 is a partial cross-sectional view of humeral head prosthesis 200 having head 202 and stem 204. Humeral head prosthesis 200 can be implanted into humerus 210. A portion of humerus 210 is cut-away in FIG. 2 to show stem 204 and intramedullary canal 220. In examples, humeral head prosthesis 200 can comprise the prosthetic humeral head system described in Pat. No. US 10,925,738, titled "Adjustable Orthopedic Connections" to Nathan A. Winslow et al. Though the present application is discussed with reference to bones of a shoulder joint, the systems, devices and methods of the present disclosure can be used to in conjunction with other bones and joints, such as ankle, knee and hip joints.

Humerus 210 can comprise humeral head 212, tubercle area 214 and diaphysis region 216. Humerus 210 can have a hard exterior formed of cortical bone and a softer interior formed of cancellous bone. Humeral head 212 can be resected to form cut surface 218 that can expose intramedullary canal 220. Humeral head prosthesis 200 can be attached to humerus 210 via insertion of stem 204 into intramedullary canal 220 until head 202 contacts or is in close proximity to cut surface 218.

Head 202 and stem 204 can be fabricated of typical materials for prosthetic implants, such as titanium or stainless steel. Such materials can be hard and as such are desirable to reduce wear and prevent damage or corrosion. However, such hard materials can be significantly harder than the bone material to which they are attached. As such, there is the potential for stem 204 to damage humerus 210, particularly during the implant procedures. To implant humeral head prosthesis 200, intramedullary canal 220 can be reamed to produce a cavity to receive stem 204. The cavity can be produced to be slightly smaller than stem 204 in order to obtain a tight fit so that humeral head prosthesis 200 is not loose and likely to shift position. Thus, in order to implant and fully seat humeral head prosthesis 200, it can be useful to impact humeral head prosthesis 200 with a force to overcome resistance of the bone. Examples of devices for delivering such force include, hammers, mallets and the like. However, such force can potentially cause unintended modification of humerus 210. For example, humerus 210 can become cracked or damaged if impacted by too high of a force, such as one that exceeds the stress limitations of the bone. Areas of a bone that have density lower than healthy bone can be especially susceptible to damage, particularly when the surgeon is unaware of bone at the impact site being weaker than normal.

The surgical planning and predicting systems and methods of the present disclosure can analyze patient-specific bone density information to help select a prosthesis and determine an implantation plan that reduces the potential for intra-operative and post operative bone damage and post-operative implant loosening. For some patients, the systems and methods can determine that the bone density pattern of a specific patient is not suitable for certain types of implants. For example, it may be that the bone matter of a specific patient may not be conducive for receiving stem 204. As such, the planning and predicting systems and methods of the present disclosure can determine that a stemless humeral head implant is desired.

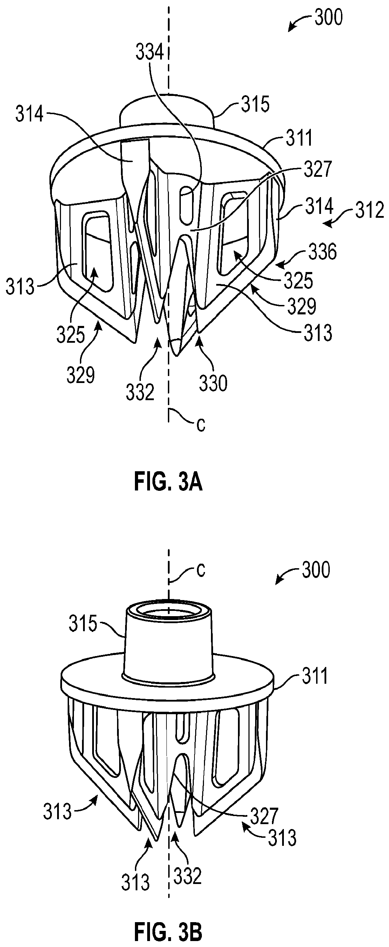

FIG. 3A is a perspective view of humeral head prosthesis 300. Humeral head prosthesis 300 can comprise a stemless humeral head prosthesis. Humeral head prosthesis 300 can comprise base plate 311 and blades 313. In examples, humeral head prosthesis 300 can comprise the humeral head implant described in Pat. No. US 8,992,623, titled "Shoulder Prosthesis" to Andrew Hopkins et al.

Humeral head prosthesis 300 can comprise base plate 311, which can comprise a circular disk having a first proximal side and a second distal side connected by an edge surface or rim. Connection portion 315 can extend from the first side of base plate 311 in a first direction and blades 313 can extend from a second side of base plate 311 in a second direction. Connection portion 315 can comprise a stud or receptacle for attaching a prosthetic humeral head (e.g., head 202 of FIG. 2) to base plate 311. In examples, connection portion 315 can comprise a tapered cylinder-like body. However, connection portion 315 can comprise any suitable connection means to reliably connect the humeral head to humeral head prosthesis 300.

The distal side of base plate 311 can be provided with anchoring means 312 that serve to reliably anchor humeral head prosthesis 300 in the humerus bone of a patient. Anchoring means 312 can comprise blades 313 extending from base plate 311. In examples, four blades 313 can be used. However, in other examples, a greater number of blades 313 or a lesser number of blades 313 can be used. In examples, blades 313 can comprise ribs or shanks having planar sidewalls that extend perpendicularly from base plate 311. In examples, blades 313 can be evenly distributed in a circumferential direction around central axis C. In examples having four blades 313, the angle between neighboring blades 313 can be 90°. However, other angles can be provided between neighboring blades 313 and the angles between all blades 313 need not be equal. Blades 313 can extend in a radial direction from central axis C. In examples of the present disclosure, the configuration of prosthetic implants can be customized for specific patients. For example, the number and orientation of blades 313 can be selected based on bone density information of a specific patient.

Blades 313 can be provided with openings 325. Openings 325 can improve blood circulation and osseointegration of humeral head prosthesis 300. Moreover, openings 325 and space F can help minimize the size of humeral head prosthesis 300 thereby minimizing the surgical impact of humeral head prosthesis 300 while at same time promoting osseointegration.

The radially inner ends 330 of neighboring blades 313 can be connected by webs 327. Webs 327 can extend distally from base plate 311 and can have lengths shorter than blades 313. Webs 327 can enclose central space 332 adjacent to base plate 311 that is free of protrusions. Therefore, in an implanted state of humeral head prosthesis 300, material of the humerus bone can extend into space 332 promoting osseointegration. To foster this process, webs 327 can be provided with openings 334 which can, among other things, improve blood circulation in regions adjacent to humeral head prosthesis 300. In particular, openings 334 can allow circulation of blood into and out of the bony material disposed in free central space 332.

The radially outer ends 336 of blades 313 can be provided with wings 314 that extend in in a circumferential direction. In examples, wings 314 can be disposed flush with the outer contour of base plate 311. Wings 314 can improve anchoring properties of blades 313 and contribute, as with webs 327, to the stability and stiffness of humeral head prosthesis 300.

The distal edges of wings 314 and blades 313 can form cutting edges 329 that can facilitate implantation of humeral head prosthesis 300. Distal edges of webs 327 are illustrated as not having cutting edges, but can be provided with cutting edges in other examples.

The geometry of blades 313 can be such that humeral head prosthesis 300 resembles, in a side view, the shape of an arrowhead, i.e. the distal edges of blades 313 can recede in a radial direction towards the radially outer end 336 of blades 313. The edges of radially inner ends 330 of blades 313 can be inclined with respect to central axis C. In other words, the radially inner edges of blades 313 diverge when viewed along central axis C from a distal surface of base plate 311, i.e., when viewed from proximal to distal. Free central space 332 can, therefore, have a conical shape tapering towards base plate 311.

The present disclosure can provide surgical planning systems and methods that can allow a surgeon to evaluate the implantation of a plurality of prostheses, including humeral head prosthesis 200 of FIG. 2 and humeral head prosthesis 300 of FIGS. 3A and 3B. For example, humeral head prosthesis 200 can have stem 204 that can occupy space extending centrally from head 202, while humeral head prosthesis 300 can be configured to preserve bone positioned centrally at head 202 via the presence of free central space 332. As such, it can be advantageous to utilize humeral head prosthesis 300 if the anatomy of the patient includes healthy bone. However, if the specific patient is involved in high-use or high-impact usage of their shoulder joint, it may be beneficial to utilize humeral head prosthesis 200 regardless of the bone density pattern.

Humeral head prosthesis 200 (FIG. 2) and humeral head prosthesis 300 (FIGS. 3A and 3B) can have different bone matter footprints and can therefore require different modification be made to humerus 210 (FIG. 2). Humeral head prosthesis 200 and humeral head prosthesis 300 will therefore interact with different bone matter of humerus 210, which can potentially have different densities. The differing bone matter density at different locations within humerus 210 can affect how well each of humeral head prosthesis 200 and humeral head prosthesis 300 will attach to bone and how well each will stay attached post-operatively, particularly based on the activity of each specific patient. The systems, devices and methods of the present disclosure can determine bone density of bone matter, simulate various outcomes for different types and shapes of prosthetic implants that engage with the bone matter, and provide evaluation and objective feedback, such as in the form of visual indicators, regarding how well the selected prosthetic implant will stay affixed to the bone matter and the susceptibility of the selected implant to specific risk factors.

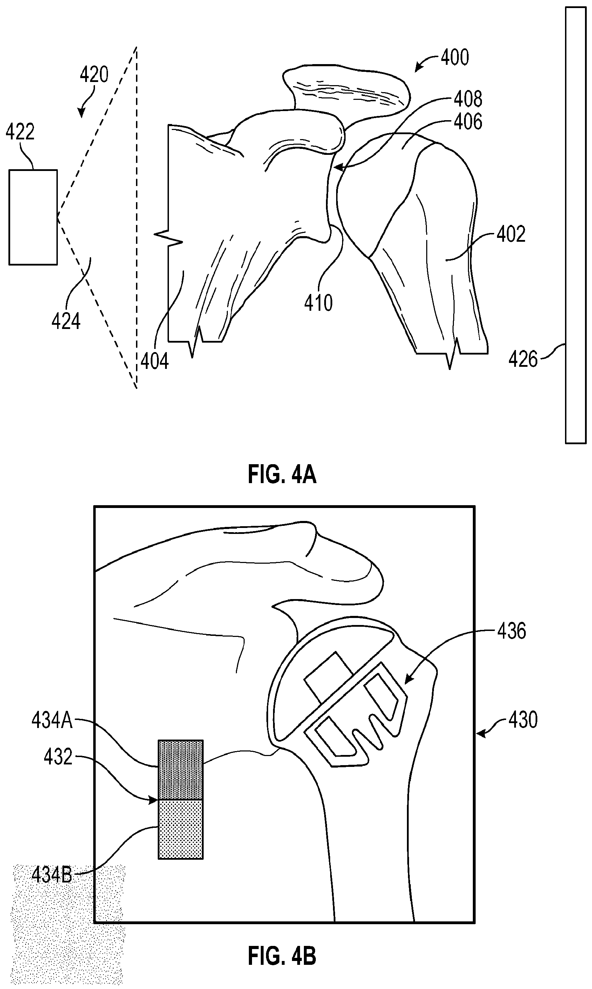

FIG. 4A is a schematic view of shoulder joint 400. Shoulder joint 400 can be of patient 110 of FIG. 1. Shoulder joint 400 can comprise humerus 402 and scapula 404. In FIG. 4A, shoulder joint 400 is illustrated proximate imaging system 420, which can comprise image generator 422, imaging projection 424 and detector 426. FIG. 4B is a schematic view of image 430 of shoulder joint 400 along with bone density scale 432 and prosthesis template 436. FIGS. 4A and 4B are discussed concurrently.

Humerus 402 of shoulder joint 400 can include head 406, which can have a general ball-shape. Scapula 404 of shoulder joint 400 can include socket or glenoid 408 having glenoid surface 410. During movement of shoulder joint 400, head 406 of humerus 402 can articulate within glenoid 408 of scapula 404 against glenoid surface 410. Though the present application is discussed with reference to bones of shoulder joint 400, the systems, devices and methods of the present disclosure can be used to in conjunction with other bones and joints, such as ankle, knee and hip joints.

In examples, imaging system 420 can comprise an x-ray imaging system wherein image generator 422 can comprise a high voltage generator x-ray tube, imaging projection 424 can comprise an x-ray beam or field, and detector 426 can comprise an imaging detector, which typically comprises a digital video detector, a solid state detector, or x-ray film. However, other types of imaging systems can be used. In examples, Magnetic Resonance Imaging (MRI), computed tomography (CT) scans, and ultrasound imaging systems can be used.

Humerus 402 and scapula 404 can be positioned proximate imaging system 420 within the field of view of image generator 422. Imaging projection 424 emanating from image generator 422 can travel through humerus 402 and scapula 404 to impinge upon detector 426. X-ray image 430 (FIG. 4B) produced by detector 426 can then pass through an amplifier and a computer for processing, or may be recorded on x-ray film in a developer.

In examples, a bone density marker, or a calibration phantom, that can replicate density of one or both of healthy and diseased bone can be positioned with the field of view of imaging system 420. In examples, bone density scale 432 can be generated with a calibration phantom. Thus, known bone mineral densities of a calibration phantom can be used to compare images of a calibration phantom to images of bone to extrapolate actual or estimated bone mineral density. In examples, a calibration phantom can comprise a block of material fabricated from a demineralized bone matrix composite. In examples, the demineralized bone matrix composite can be made according to Pat. No. US 7,582,309 to Rosenberg et al., which is assigned to Etex Corporation. In examples, the demineralized bone matrix composite can be fabricated from Equivabone^{®} Bone Graft Substitute or βBeta-bsm^{®} Injectable Bone Substitute Material, each of which is commercially available from Zimmer Biomet. In other examples, bone density scale 432 can comprise a graphic image or icon derived from empirical data that can be included on x-ray image 430. In additional examples, a bone density calibration phantom can be used, such as those commercially available from QRM.

Image 430 of shoulder joint 400 can show various internal features of humerus 402 and scapula 404, such as soft tissue, cartilage and bone density. Humerus 402 and scapula 404 can have varying bone density from patient to patient, which can be visible in x-ray images and other image types. Bone density is a product of bone mineral content in the material forming the bone. Bone density can be indirectly determined or estimated by measuring the density per square unit of bone within an x-ray image. As such, healthy bone will have a density, e.g., an associated grayscale intensity in image 430, replicated by shading 434A on scale 432, while diseased or damaged bone will have a different, e.g., less dense, density, which can show up in image 430 as a lighter grayscale intensity, represented by shading 434B on scale 432. For example, bone matter afflicted with osteoporosis can be less dense than healthy bone and can show up as a lighter grayscale intensity than healthy bone in an x-ray image. Less dense bone can be weaker than healthy bone and is, therefore, more vulnerable to breaking or cracking. For example, hip replacement procedures are particularly vulnerable to bone cracking, either directly from the implant procedure or due to long term fatigue that arises from stresses imparted during the implant procedure. Thus, reduction in the amount of stress imparted to a bone during an implantation procedure can be used to reduce the occurrence of revision surgeries.

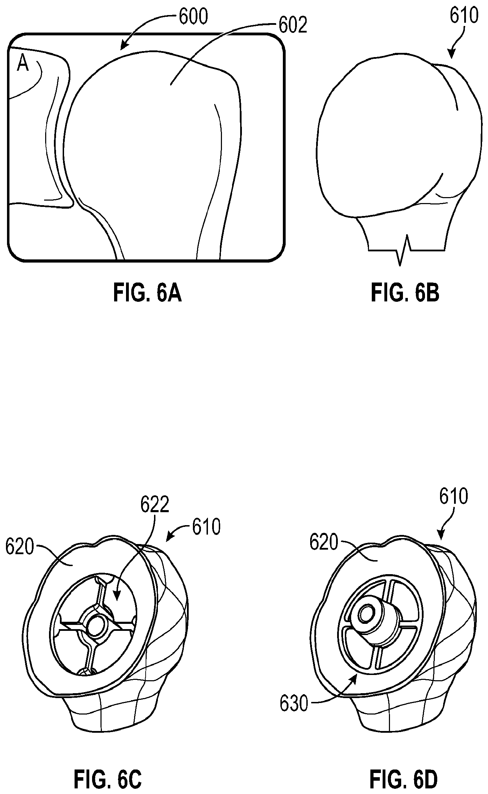

As is discussed in greater detail below, particularly with respect to FIGS. 6A - 6G, imaging of shoulder joint 400 can be used to generate patient-specific, three-dimensional models that can include numerical modeling. Bone modifications and a particular prosthesis can be incorporated into the three-dimensional models such that the numerical models can be solved to generate biomechanical data, such as, for example, bone fixation or implant resistance data for different scenarios. The scenarios can thus be evaluated, either automatically via a computer system or manually via a surgeon, to select an implant and determine an implantation plan.

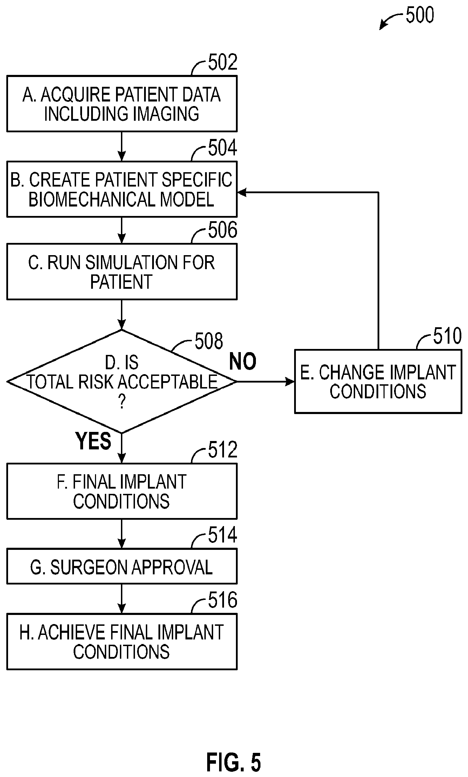

FIG. 5 is a block diagram illustrating steps of method 500 for planning and performing a surgical procedure using patient-specific bone density information to predict prosthesis fixation.

At step 502, patient data can be collected from a patient, such as patient 110 of FIG. 1. Patient information such as, age, gender, height, weight, activity level, ethnicity, diagnoses (e.g., health conditions) and the like can be collected from the patient. This biographic patient information can be stored in a computer readable medium accessible to computing system 140 (FIG. 1).

Imaging can also be collected from the same patient, including from various two-dimensional and three-dimensional sources including ultrasound, CT scan, MRI, and X-ray. The image files can be stored in a computer readable medium accessible to computing system 140 (FIG. 1). A plurality of images of one or more joints and bones of a patient can be obtained. The anatomy can be imaged a plurality of times in different orthogonal directions, e.g., anterior, posterior, medial and lateral. A calibration phantom or bone density marker, as mentioned above, can be positioned within each image. One calibration phantom can be used in all of the images or a plurality of calibration phantoms each having the same construction can be used. However, different types of calibration phantoms can be used so long as the same or consistent bone mineral density information can be obtained and used in a normalized fashion. In examples, CT images are used because bone density information can be readily extrapolated from CT images, particularly when used with calibration phantoms. However, bone density may be determined from other imaging techniques, such as by using ultrasound densitometry, X-ray or MRI.

This data, particularly the bone density information, can then be used to produce and inform a numerical patient-specific model that can assess how well anchoring means of various prostheses will stay attached to specific bone matter that engages each prosthesis.

At step 504, a patient-specific model of the patient data can be generated. The patient-specific model can include biomechanical and kinematic data, such as various axes of the joint and the like. Three-dimensional models can be produced from the two-dimensional and three-dimensional imaging previously obtained at step 502. The three-dimensional models can represent virtual, digital models of anatomy of the patient. The patient-specific models can comprise numerical models wherein the bone matter is assigned a plurality of numerical points in 3D space that can be assigned as variables in one or more mathematical equations that represent the bone matter. The numerical points can be assigned values, such as based on the stiffness or density or strength of the bone matter at that three-dimensional location in the model, as can be determined from analysis of the imaging. Further details of the production and generation of patient-specific models is described with reference to FIGS. 6A - 6G. In any event, the patient-specific model of the patient can include appropriate scaling of bones in a joint, the physical relationship of the bones in a joint and bone density information for each bone in a joint. As such, the patient-specific model of the patient can comprise a virtual representation of the real-world joint from which simulations can be run to simulate prosthesis implant conditions and usage conditions.

Bone density information can be determined from the imaging and incorporated into the patient-specific model of the patient. For example, bone density of each bone in the plurality of images can be determined using density markers or calibration phantom placed in the scanner. For example, grayscale intensity of the bones in each image can be compared to grayscale intensity of the bone density marker or calibration phantom to match areas of the bone to specific bone mineral densities represented by the bone density marker. In another example, bone density of each bone in the plurality of images can be determined using known density of materials in the image, such as air, fat, muscle etc., a method known as phantomless calibration. Bone density information from the different orthogonal views can be pieced together during construction of three-dimensional models for each bone. In examples, bone density information can be determined directly from grayscale intensity in the images by computer assessment of the shading in the images. As such, grayscale intensity in the images can represent healthy or unhealthy bone, strong or weak bone and the like. The compilation of images from different angles can allow for three-dimensional plotting of bone density throughout the bone. In examples, piecing together of images to formulate bone density information can be conducted automatically via the CT scans. Examples of bone density markers and calibration phantoms are described in Pat. No. US 10,736,601 to Kopperdahl et al., titled "Quantitative phantomless calibration of computed tomography scans.

In examples, the three-dimensional model can comprise a mean bone model or a statistical shape and intensity (SSIM) model into which patient-specific bone density information can be imparted. The bone model or mean bone can be a generalized model based on multiple patient bone models and can be selected from a principle component analysis ("PCA") based statistical bone atlas. One such a priori bone atlas, formed in accordance with method 500 includes a dataset of four-hundred dry femur and tibia bone pairs, scanned by CT and segmented to create models of each bone. The method of building and using one such statistical atlas is described in MAHFOUZ M et al., "Automatic Methods for Characterization of Sexual Dimorphism of Adult Femora: Distal Femur," Computer Methods in Biomechanics and Biomedical Engineering, 10(6) 2007. Further description of such a bone model and associated procedures for generating a bone model are described in Pat. No. US 10,512,245 to Mahfouz titled "Method and apparatus for three-dimensional reconstruction of a joint using ultrasound,". Additional examples of generating mean bone models are described in Pat. No. US 10,130,478 to Mahfouz titled "Ethnic-specific orthopaedic implants and custom cutting jig,". See Incorporating Population-Level Variability in Orthopedic Biomechanical Analysis: A Review by Bischoff et al., Journal of Biomechanical Engineering, FEBRUARY 2014, Vol. 136 / 021004-1, for further description of statistical shape and intensity models.

In examples, bone density patterns can be incorporated into three-dimensional bone models to, for example, provide an indication of where a bone is relatively stronger and relatively weaker. Such information can be used to plan procedures for implanting orthopedic devices.

In a first example, the bone density pattern of the specific patient can be incorporated into a three-dimensional model of that patient's bone. In a second example, the bone density pattern of the specific patient can be incorporated into a mean bone model having dimensions of a mean bone. In such examples, the specific patient's bone density information can be directly analyzed. For example, a virtual implant can be positioned in the patient-specific bone matter and biomechanical simulation analysis (e.g. primary stability, stress shielding), can be performed.

In a third example, the bone density pattern of the specific patient can be compared to bone density patterns of a mean bone model having mean bone density information. In a fourth example, the bone density pattern of the specific patient can be compared to bone density patterns in a database of an artificial intelligence or machine-learning engine. In such examples, the specific patient's bone density information can be compared to aggregated bone density information of a fixed or changing population of patients to first identify areas of healthy and unhealthy bone before simulating the implant of a prosthesis. Such comparisons to bone density information of a population can expedite or speed up the simulation process to initially eliminate untenable prosthesis selection and placement before running simulations.

At step 506, simulations can be run based on the patient-specific model, biomechanical data of step 504, and/or patient data of step 502. The simulations can comprise a plurality of different scenarios. The scenarios can include: 1) a simulated selected prosthesis, 2) a simulated cut plane as well as any other cuts or bone modifications for the selected prosthesis of 1), 3) a simulated placement location of the selected prosthesis on the bone, 4), a simulated orientation of the selected prosthesis at the simulated placement location, and 5) a simulated post-operative usage of the prosthesis. The simulations can involve analyzing the engagement of the geometry of the prosthesis with bone matter to determine, for example, how well the bone matter holds the prosthesis in place as well as risks to the bone matter from engagement with the prosthesis. Higher density bone can achieve a better fixation with a prosthesis than lower density bone, as more bone substance contacts the implant and is available to hold the implant in place.

Bone modification to accept the prosthetic implant can be identified on the patient-specific bone model. Thus, resection planes and bone modifications to accept fixation features can be directly plotted on the patient-specific model of the patient to accommodate weak or low-density bone, such as by plotting resection planes to remove weak bone or avoid weak bone or to ensure that bone anchors or stems can be adequately fixed to bone. In examples, bone modification plotting can be electronically drawn directly onto the patient-specific model of the patient using graphical user interface tools, such as a mouse to manipulate resection planes in a graphical user interface (e.g., a video display device).

Once the patient-specific model of the patient has been properly modified to accept a virtual prosthesis, simulations can be run on the patient-specific model of the patient by solving equations related to the finite element analysis. The model can apply force data and material data (e.g., bone density) and simulate the biomechanical behavior of the system. As such, loading on various surfaces and portions of the bones in a joint can be simulated to determine the risk level for various factors, such as bone or implant breakage and implant loosening.

In examples, stability of an implanted prosthesis can be determined as is described in Favre P, Seebeck J, Thistlethwaite PA, Obrist M, Steffens JG, Hopkins AR, Hulme PA. In vitro initial stability of a stemless humeral implant. Clin Biomech (Bristol, Avon). 2016 Feb;32:113-7. doi: 10.1016/j.clinbiomech.2015.12.004. Epub 2015 Dec 21. PMID: 26747397.

As discussed in greater detail below, artificial intelligence and machine learning can be utilized to inform the simulations, such as by training the software to recognize areas of weak bone density and plan appropriate prosthesis selection and bone modifications, e.g., resections, that avoid placement of the prosthesis in weak bone and weakening the bone. For example, bone density information from the patient population used to generate various patient-specific models of various patients over time can be used to identify known areas of healthy and weak bone density and what associated healthy and weak bone areas look like in imaging. Computing system 140 can then review the patient-specific model of a particular patient to identify similar patterns in the imaging that resemble the healthy and weak bone areas to thereby find a prosthesis having fixation features that will engage as much healthy bone as possible or as desired to meet certain risk criterion. Thus, a patient-specific bone density pattern can be compared to a library of bone density patterns or amalgamations of bone density patterns to help identify areas of weak bone. The patient-specific bone density pattern can then be incorporated into the bone density library and amalgamations for future use with other patients.

As discussed below, the simulations can solve equations to determine, for example, the fixation between the prosthesis and bone matter. Finite element simulations can quantify the relative motion at the bone-implant interface generated in performing an activity and compare this motion against maximum thresholds to evaluate the likelihood that the prosthesis can or will become loosened as a result of performing the activity, either directly or over time. The simulations can additionally solve mechanics of materials equations to determine, for example, the strength of bone where modifications have occurred to determine the likelihood of the bone cracking or breaking during the implantation procedure or post-operatively during use by the patient.

At step 508, risks associated with each of the different scenarios of step 506 can be assessed. The mathematical equations associated with the numerical models for the selected scenario can be solved. The mathematical equations can provide numerical output relating to the frictional engagement between a selected prosthesis implanted into the specific patient's bone matter in comparison to expected loading on the patient's bone and the prosthesis during use. As such, the evaluation can comprise a comparison of friction, force, stress and strain analysis from the simulations to threshold friction, force, stress and strain data. The threshold friction and force data can comprise levels at which a prosthesis will become loosened or not have sufficient holding power to stay attached to bone matter of the life of the patient. The threshold stress and strain data can comprise levels at which the patient's bone or implant can become fractured or broken or might become compromised from normal wear and tear imposed by the patient or by particular activities the patient might undertake based on collected biographical information. The numerical output can be compared to the threshold levels to determine a probability of the prosthesis will be come dislodged or the bone or implant will become damages.

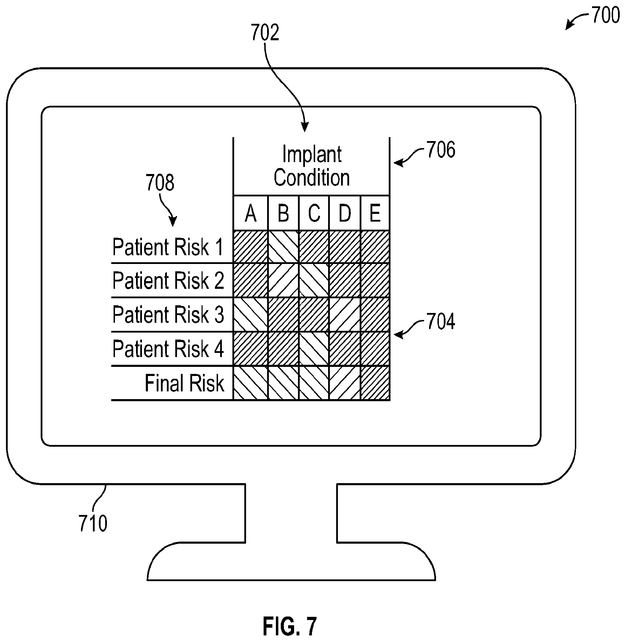

The mathematical equations can output feedback to the surgeon in different forms relating to the different risk factors, such as loosening of the selected prosthesis, stress shielding (resorption of the bone due to the larger stiffness of the metal of the prosthesis) of the bone matter for the selected prosthesis, dissociation for a device (wherein the prosthetic head and the stem or anchor can become separated), and the like. The methods and systems of the present disclosure can automatically evaluate the risk factors to determine if the simulated scenario is adequate. In additional examples, the surgeon can manually evaluate the risk factors to determine if the simulated scenario is adequate. The risk factors can be evaluated in total, where more risk might be acceptable in one category if the risk in other categories is low. Additionally, a single risk factor can be selected as being controlling as long as other risk factors are not high. Risk factors can be presented in visual form, as is discussed with reference to FIG. 7.

If the risks for a selected scenario are determined unacceptable, method 500 can proceed to step 510. For example, if the simulated or predicted friction, force, stress and strain data exceeds threshold limits for these values, the selected scenario can be deemed unacceptable.

If the risks for a selected scenario are determined acceptable, method 500 can proceed to step 512. For example, if the simulated or predicted friction, force, stress and strain data does not exceed threshold limits for these values, the selected scenario can be deemed acceptable.

At step 510, the implant conditions for the scenario can be changed or a different prosthesis can be selected. For example, different cut planes or the location of the prosthesis in a cut plane can be changed. Additionally, the prosthesis can be customized to achieve better risk scenarios, such as by changing the shape or geometry of the selected implant or other implants to better engage bone of a specific patient. For example, the number and orientation of blades 313 (FIGS. 3A and 3B) can be selected based on bone density information of a specific patient. New numerical models can be created or produced based on the updated scenario, and the new numerical models can be solved for the different scenario and the risk for the new scenario can be evaluated at step 508. Thus, steps, 506, 508 and 510 can be repeated until the system of a surgeon determines that a scenario has been found where the risks are acceptable.

At step 512, the implant conditions for the scenario can be finalized. In examples, risk results of the approved scenario can be compared to other scenarios. In order to identify those implant conditions that have the highest potential to deliver the best clinical outcome for a given patient, several plausible implant conditions can be generated for comparison. This can be done by generating random implant conditions, or in a more controlled manner (with for example Design of Experiment (DOE)) to generate a smaller but still meaningful number of different implant conditions. Once an acceptable configuration has been achieved, this implant condition can be considered final. At this point, the search for an appropriate implant condition can be stopped. However, there may exist several very different but still acceptable implant conditions, based on the different risk factors, for example, so other acceptable solutions can be found by computing system 140 that can be provided to the surgeon.

At step 514, a surgeon can approve the final implant conditions. The surgeon can take the final decision and chose a preferred option based on parameters that are not included in the simulation, such as for example, surgeon preferences, surgeon experience, etc.

At step 516, final implant conditions can be achieved. Thus, the selected prosthesis and the implant conditions, e.g., cut plane and location on the cut plane, can be saved in computer readable medium accessible to computing system 140 (FIG. 1). Once the final implant condition is validated by the surgeon, the plan for implantation according to the final implant condition can be prepared. This can include, but is not limited to, preparing surgical guidelines (as a written report, or as augmented reality (AR) or virtual reality (VR) display), surgical guide instruments (patient specific instrumentation (PSI) such as a jig), robotic surgery plan and the like.

FIGS. 6A - 6G illustrate a sequence of steps in prosthesis fixation modeling systems and methods of the present disclosure. FIGS. 6A - 6G represent examples steps that can be used and more or fewer steps can be executed.

FIG. 6A shows an x-ray image of shoulder joint 600 including humeral head 602. A plurality of images of shoulder joint 600 from different angles can be obtained. A sufficient number of images can be obtained to determine three-dimensional bone density throughout shoulder joint 600 and humeral head 602. In examples, a CT scan of patient 110 (FIG. 1) can be used to segment humeral head 602 by extracting the bony outline of humeral head 602 on each scan image so that the views can be pieced together to obtain a three-dimensional model with three-dimensional bone density information. As mentioned, x-ray and MRI imaging can also be used.

FIG. 6B shows bone model 610 of humeral head 602 of FIG. 6A. The outline of humeral head 602 from various images can be connected to reconstruct the 3D bone geometry of humeral head 602. 2D X-rays can also be used to generate a 3D model by using 2D to 3D algorithms. The 3D bone density can be extracted from the grayscale intensity of each pixel, for each scan image. Each pixel can be assigned a coordinate (x, y, z) in three-dimensional space relative to a common origin.

FIG. 6C shows cut surface 620 of bone model 610 of FIG. 6B. Cut surface 620 can include template 622. The 3D bone geometry on bone model 610 can be virtually cut. The depth D of the cut relative to the apex of humeral head 602 can be selected by a surgeon or by computing system 140 and thus can vary in different scenarios. Resection of bone model 610 can produce cut surface 620. Template 622 can be placed on cut surface 620. Template 622 can comprise a representation of the imprint that a selected prosthesis will make in cut surface 620 when implanted. Template 622 can comprise three-dimensional information having (x, y, z) coordinates, wherein (x, y) coordinates can be on cut surface 620 and a (z) coordinate can represent depth into cut surface 620. For different scenarios, the (x, y) coordinates for template 622 from the edges of cut surface 620 at the cortical bone of humeral head 602 can vary. The cut depth D and (x, y) placement of template 622 can vary in different scenarios (e.g., step 504 of FIG. 5), and can be selected to avoid or remove weak or unhealthy bone, for example, and increase the frictional engagement and/or support of the prosthesis. Thus, as shown in FIG. 6D, a virtual representation of the selected prosthesis can be placed onto cut surface 620.

FIG. 6D shows virtual prosthesis 630 inserted into template 622 of cut surface 620 of FIG. 6C. The 3D bone geometry of humeral head 602 can be cut and prosthesis 630 can be placed during virtual surgery. Such virtual placement of prosthesis 630 can allow a surgeon to visually inspect the placement. In examples, FIG. 6D can be viewed on interface devices 145 (FIG. 1) and can be three-dimensionally rotated by the surgeon for inspection of the placement.

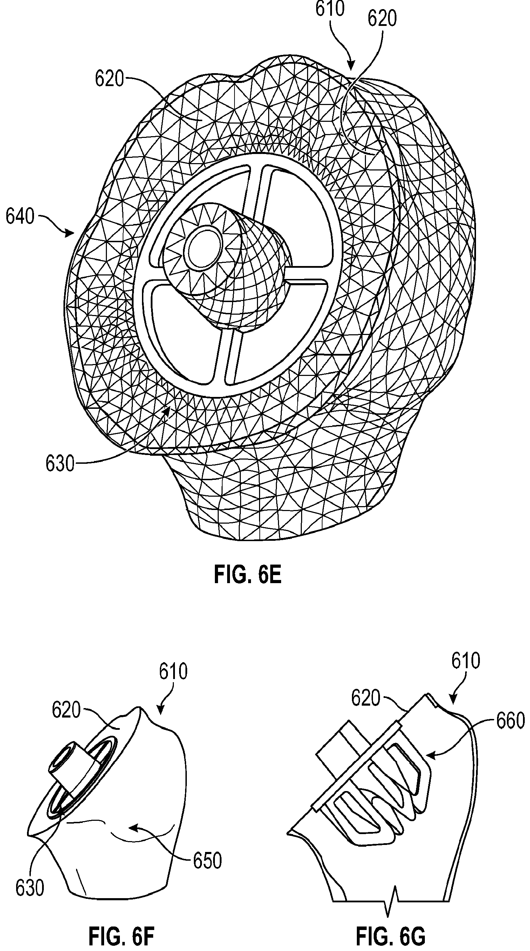

FIG. 6E shows bone model 610 and prosthesis 630 of FIG. 6D discretized into mesh 640 of finite elements. Model 610 and prosthesis 630 can be discretized into finite elements each comprising an element and nodes, numerical value or three-dimensional (x, y, z) coordinate. The finite elements can comprise inputs to variables within mathematical formula representing humeral head 602.

FIG. 6F shows simulated force 650 applied to prosthesis 630 of FIG. 6E. Force 650 as well as other forces can be applied to prosthesis 630 to simulate physical activity. Physical activity can be simulated based on published reaction force data, which can be scaled based on patient-specific information, such as weight, or from musculoskeletal modeling. In examples, reaction force data can be obtained from databases that include loads acting on joints for routine and sportive activities, such as the publicly available database available from Orthoload of the Julius Wolf Institute. In additional examples, musculoskeletal models (such as, for example, OpenSim or Anybody) can be used to obtain muscle and reaction force data.

In examples, patient-specific information can be collected from a data tracker that the patient can wear and that can transmit data to the cloud or the internet for eventual transmission to computing system 140 (FIG. 1). In examples, the data tracker can be used with MyMobility^{®} software commercially available from Zimmer Biomet. The data tracker can record speed and motion data generated by the patient that can then be translated into loading information on the bones using specific algorithms.

Simulated force 650 can comprise a vector indicating magnitude and direction of the sum of forces on prosthesis 630. Simulated force 650 can be applied to the numerical equations describing bone model 610 and prosthesis 630 in order to, for example, solve for resulting loads on the bone matter of humeral head 602.

FIG. 6G shows prosthesis 630 of FIG. 6F rendered as visual indicator 660 to provide risk assessment feedback. The models of prosthesis 630 and humeral head 602 can be solved to assess the performance of prosthesis 630 under the given conditions, e.g., force 650 of FIG. 6F, for a given output of interest, e.g., a risk factor. Depending on the type of prosthesis, the specific information of the patient, and known major failure modes, the results can be focused on one output of interest (ex. loosening for a stemless uncemented device), or include several outputs of interests (ex. loosening, stress shielding, dissociation for a stemmed device).

In examples, patient-specific model 610 can be another type of model than the Finite Elements models described with reference to FIGS. 6A - 6G. For example, mechanical issues can be simulated by means of FE, but depending on the studied patient risk, another model form can be more favorable. For example, the simulation of range of motion (ROM) can be achieved with less computationally expensive modeling approaches (ex. CAD, rigid body simulation). Also, to provide a more complete assessment, different computational approaches can be combined, for example a ROM and FE patient-specific model.

The simulated loading on prosthesis 630 can be represented with a color-coded representation. The color-coding can represent forces on prosthesis 630, such as from bone (e.g., frictional engagement) of from bone as transmitted from impact data. Likewise, though not illustrated in FIG. 6G, model 610 can be rendered as a color-coded representation to visualize loading of the bone to assess the potential for bone damage.

The color coding of prosthesis 630 and model 610 can be directly evaluated by a surgeon or surgical planner. In examples, the color coding of prosthesis 630 and model 610 can be evaluated by computing system 140 (FIG. 1) to output specific risk levels and recommended implant scenarios, as is discussed with reference to FIG. 7.

FIG. 7 is a schematic view of display screen 700 for surgical system 100 showing visual output 702 for evaluating orthopedic implant fixation using bone density. Visual output 702 can comprise matrix 704 of implant conditions 706 and patient risks 708. Implant conditions 706 can be provided in columns labeled A, B, C, D and E. Patient risks 708 can be provided in rows labeled 1, 2, 3, 4 and Final. Visual output 702 can be displayed on video monitor 710.

Matrix 704 can comprise individually colorable squares or indicators that can be color-coded for a risk condition. In examples, green can indicate that risks are acceptable or that the risk is negligible or not present (e.g., the aforementioned thresholds are not met), yellow can indicate that risks are possibly present, but at levels below concern (e.g., the aforementioned thresholds are close), and red can indicate that risks are present and at levels of concern, such as levels where a failure mode may occur (e.g., the aforementioned thresholds are exceeded). Green, yellow and red can be substituted for any colors, including black, grey and white or a greyscale.

Patient risks 1 - 4 can comprise one or more of implant loosening, bone fracture, implant fracture, stress shielding, bone removal, cortex perforation, etc. Additionally, more or fewer patient risks can be considered. Implant conditions A - E can comprise one of the scenarios previously discussed where a particular prosthesis is implanted at a particular cut depth and at a particular location on the cut plane. Additionally, more of fewer implant conditions can be considered. Implant conditions A - E can comprise randomized scenarios for comparison to desired scenarios as a reference check.

If several different patient risks are investigated, prosthesis 630 can be considered to perform well only if the final risk is acceptable for all outputs. For example, if a considered condition leads to a higher than acceptable level for one (condition A) or more patient risks (condition C), the final risk for the patient is high. If a considered condition leads to a medium level for one patient risk (condition D), the final risk for the patient is medium. If a considered condition leads to a medium level for one patient risk and high for another patient risk (condition B), the final risk for the patient is high. If a considered condition leads to a low level for all patient risks (condition E), the final risk for the patient is low, and this position can be considered the most appropriate.

The patient conditions A - E and the associated risks 1 - 4 can be saved for use in training machine learning software.

Matrix 704 can comprise an example of visual indicators that can be provided to a user. Other visual indicators can be provided, such as numeric values, a sliding scale, dials and the like. Additionally, audio warnings can be provided alone or in combination with visual indicators.

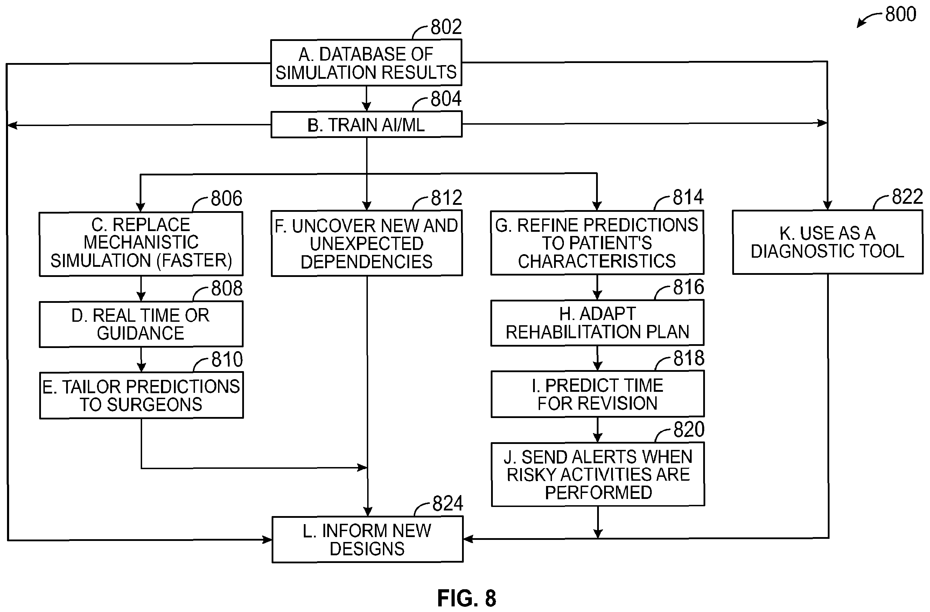

FIG. 8 is a block diagram illustrating steps of methods 800 for training an artificial intelligence engine for running prosthesis fixation simulations.

At step 802, a database of simulation results can be maintained. For example, patient conditions A - E and the associated risks 1 - 4 of FIG. 7 can be stored in a database for use to train software to develop patient conditions for a different patient. The database can be maintained in a computer readable medium accessible to computing system 140 (FIG. 1). The database can include the biographic patient information (disassociated from any patient identifying information, such as name and social security number), biological information (e.g., bone size and dimensions), biomechanical information (e.g., kinematic axes and the like), the patient conditions or scenarios, the risk factors and the risk assessments (e.g., color codes) for each patient in the database.

At step 804, multiple implant conditions that have been simulated, and their corresponding performance can be leveraged to train Artificial intelligence (AI)/machine learning (ML) models. The AI and ML models or engines can be trained to identify areas of healthy and unhealthy or strong and weak bone matter within a bone. The AI and ML models can be trained to look for patterns in the bone density that can mesh with fixation features of various prostheses to result in strong fixation of the prosthesis and that avoid potential damage to the bone and loosening of the prosthesis.

At step 806, AI/ML surrogate models can then determine an optimal configuration for new patients or new use conditions, reducing or removing the need for mechanistic simulations, with the advantage that this technique can be much faster. That is, the AI and ML models or engines can directly assess a patient's bone anatomy and density to find a desirable (e.g., low risk) solution for one or more prostheses directly without having to run any simulations. Additionally, the AI and ML models can eliminate undesirable implant scenarios so that fewer simulations can be run, thereby saving time.

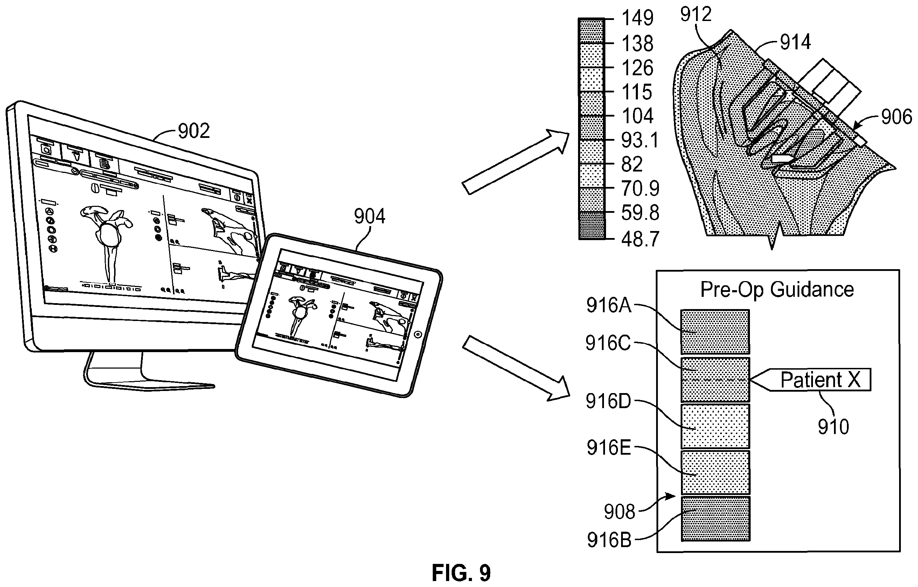

At step 808, with the faster AI/ML approach, a real time guidance/confirmation in the OR can be envisioned. Potential display solutions can include a direct representation of the output displayed on a 3D representation of the bone or as a scale (as is discussed with reference to FIG. 9), both updating in real time as the surgeon changes the implant condition or scenario. This could be projected on a screen in the operation room, or in a headset, such as by using AR/VR.

At step 810, predictions can be tailored to surgeon preferences (ex. downsize implant, free hand cut, etc.) to help refine the predictions for that surgeon. For example, if two predictions or surgical outcomes are found to have low risk or no risk and are therefore acceptable, if the system is provided with surgeon preferences, recommendations can be made accordingly, e.g., by eliminating recommending surgical techniques, prostheses or instruments the surgeon does not prefer.

At step 812, using AI/ML, the data can be mined further to uncover new and unexpected relationships and dependencies. The model can then determine an optimal configuration with sufficient accuracy based off reduced inputs (for example with demographic data only and no CT scan).

At step 814, predictions can be tailored to specific patient characteristics (comorbidities, patient deformities, patient bone quality, etc.) to help refine the indications for the different patient populations and type. Also, based on desired activity level of patient, the predictions can help intra-operative decisions on implant condition to increase chances of a successful surgery. For example, if the patient is very active, a stemmed or cemented option may be best to resist the high loads. If the patient is not very active, a more bone preserving option can be chosen.

At step 816, predictions can be used to adapt the rehabilitation plan. If the predictions are encouraging, a more aggressive plan can be implemented (ex. faster return to work). If predictions highlight some risks, the rehabilitation plan can be more conservative, follow up can be more targeted (ex. X-ray for patient at risk of early loosening) to avoid early failure.

At step 818, the time for when a revision procedure might be needed can be predicted. For example, the force and loading simulation data can predict that a particular fixation scenario can be suitable for a period of time, but that the implanted prosthesis might be ready for a revision surgery after a specified period of time. Actions can be proposed for the surgeon or the patient to increase longevity. For example, the patient can be instructed to avoid certain activities.

At step 820, the patient can be monitored for activities that can propose a risk to the patient and the implant. This can be coupled with the MyMobility^{®} application and other patient monitoring devices, implant sensors or smart cloths to measure patient activities, and send alerts if such devices sense that risky activities are being performed.

At step 822, mechanistic and AI/ML models can be used as a diagnostic tool. If the patient is in pain, a new patient specific model can be created for her/him, potential sources of the pain can be assessed, and targeted treatments can then be identified and proposed. This can be done instead of the surgeon operating to see what the source of pain may be.

At step 824, gained knowledge can be used to inform design for new improved implants. For example, different geometries of fixation features can be determined that are configured to engage with healthy bone in a larger segment of the population. For example, non-symmetric fixation bladed (FIGS. 3A and 3B) can be configured or offset stems (FIG. 2) can be configured.

FIG. 9 is a schematic view of display monitor 902 and mobile computing device 904 providing output indicia for evaluating prosthesis fixation. In examples, display monitor 902 and mobile computing device 904 can be configured to output indicia comprising color-coded prosthesis 906 and fixation feedback scale 908.

In examples, patient bone models can be displayed in a graphical user interface for manipulation by a surgeon or surgical planner. The bone models can be virtually altered to accommodate a specific prosthesis with a particular bone modification plan. Numerical models representing the virtual models can be solved to render the prosthesis as color-coded prosthesis 906. Fixation feedback scale 908 can be simultaneously provided with color-coded prosthesis 906 to decode color-coded prosthesis 906. Fixation feedback scale 908 can include indicator 910 that can provide the surgeon or surgical planner with an overall assessment of color-coded prosthesis 906 in order to reduce or eliminate the need for a person to evaluate or decode the simulation data. Color-coded prosthesis 906 can be displayed attached to bone model 912 at resected surface 914. Color-coded prosthesis 906 can comprise an instance of model 610 (FIG. 6B). Colors of color-coded prosthesis 906 can match up to colors shown in fixation feedback scale 908. Fixation feedback scale 908 can have a first color 916A that can indicate the outcome of a given scenario as having no risk and second color 916B can indicate the outcome of a give scenario as having high risk. Colors 916C, 916D and 916E can represent risk levels between those of colors 916A and 916B. In examples, color-coded prosthesis 906 and fixation feedback scale 908 can be updated in real-time using graphical user interfaces. For example, a virtual prosthesis can be moved around on a virtual model and color-coded prosthesis 906 and indicator 910 can adjust accordingly (e.g., colors and position, respectively) in real-time.

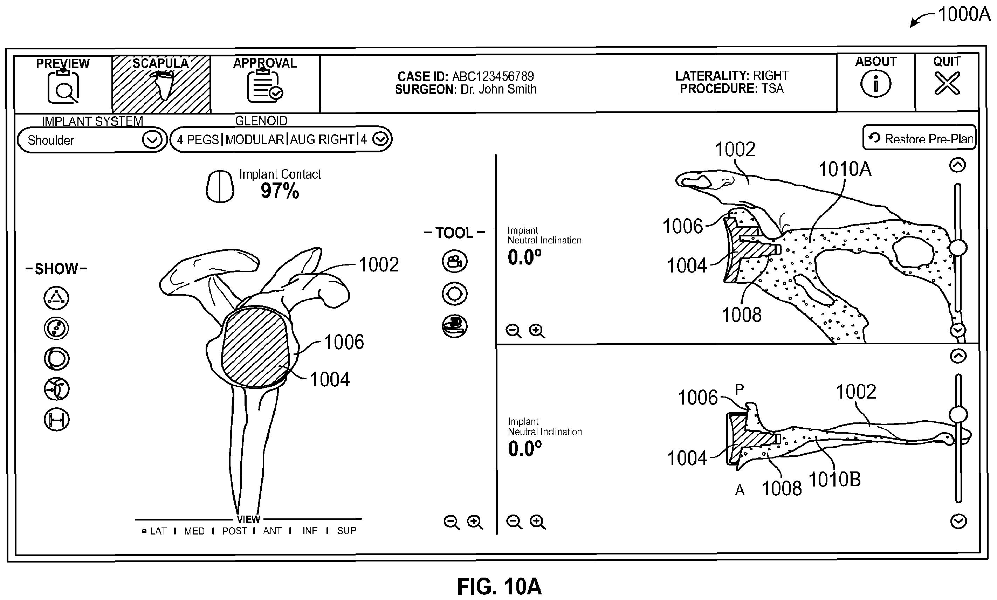

FIG. 10A is a schematic view of display screen 1000A suitable for display on display monitor 902 and mobile computing device 904 of FIG. 9. Display screen 1000A can include digital planning tools for predicting fixation of an implant. Display screen 1000A can include model 1002, implant 1004, modification surface 1006, prosthesis template 1008, first cross-section plane 1010A, second cross-section plane 1010B.

In the illustrated example, model 1002 can comprise a scapula and implant 1004 can comprise a prosthetic glenoid. However, model 1002 and implant 1004 can comprise any anatomic feature and prosthetic device, such as a humerus bone and a humeral head prosthetic. In examples, model 1002 can comprise an instance of model 610 (FIG. 6B). Modification surface 1006 can comprise a resected or reamed bone surface into which implant 1004 is to be attached. Prosthesis template 1008 can comprise indentions into modification surface 1006 to receive fixation features of implant 1004, which in the illustrated embodiment can comprise fixation pegs. Cross-section planes 1010A and 1010B can be provided in additional view of model 1002 to allow a surgeon to see the interface between model 1002 and implant 1004.

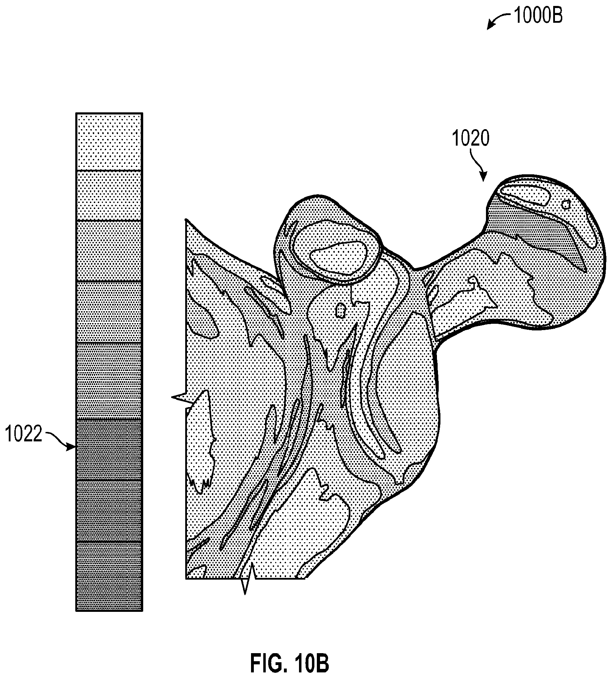

FIG. 10B is a schematic view of display screen 1000B including model 1020 placed alongside bone density scale marker 1022. Model 1020 and bone density scale marker 1022 can be displayed on a display screen 1000A in place of or in addition to model 1002. As such, FIG. 10B can include the same elements as FIG. 10A, except the isometric view of model 1002 can be replaced with a bone density representation 1020 alongside bone density scale marker 1022, but are omitted for clarity.

In examples, bone density representation 1020 can be constructed using osteoabsorptiometry as is described in von Eisenhart-Rothe R, Müller-Gerbl M, Wiedemann E, Englmeier KH, Graichen H. Functional malcentering of the humeral head and asymmetric long-term stress on the glenoid: potential reasons for glenoid loosening in total shoulder arthroplasty. J Shoulder Elbow Surg. 2008 Sep-Oct;17(5):695-702. doi: 10.1016/j.jse.2008.02.008. Epub 2008 Jun 16. PMID: 18558500.

In examples, bone density representation 1020 can be constructed using CT scans as is described in Couteau B, Mansat P, Mansat M, Darmana R, Egan J. In vivo characterization of glenoid with use of computed tomography. J Shoulder Elbow Surg. 2001 Mar-Apr;10(2):116-22. doi: 10.1067/mse.2001.112884. PMID: 11307073.

Display screen 1000A and 1000B can be used in an operating room or in pre-operative or post-operative settings to allow a surgeon to rapidly visualize risks associated with prosthesis fixation and the like for different implant scenarios. The surgeon can engage with display screens 1000A and 1000B to rotate model 1002, move the resection plane, move the location of fixation features, chose different prostheses and see how such changes affect the fixation and risk assessment in real time.

FIG. 11 illustrates system 1100 for performing techniques described herein, in accordance with some embodiments. System 1100 is an example of a system that can incorporate surgical system 100 of FIG. 1. System 1100 can include planning system 1102, surgical system 1104, display device 1106 and control system 1108. Planning system 1102 can comprise simulation engine 1110 and artificial intelligence engine 1112. Surgical system 1104 can comprise tracking system 1114 and robot system 1116. Display device 1106 can comprise graphical user interface 1118. Control system 1108 can comprise processor 1120 and memory 1122.

Tracking system 1114 can comprise tracking system 165 described with reference to FIG. 1 and can comprise tracking elements 170, cameras, registration devices and the like.

Robot system 1116 can comprise the robotic system 115 described with reference to FIG. 1 and can comprise robotic arm 120 and the like.

Display device 1106 can be used to display graphical user interface 1118 to allow an operator to receive output from system 1100 and provide input to system 1100. Graphical user interface 1118 can display three-dimensional virtual models of anatomy of a patient that include patient-specific bone density information along with a model of a selected prosthesis. Graphical user interface 1118 can allow a surgeon or surgical planner to manipulate the models to plan a surgical procedure. Graphical user interface 1118 can provide feedback on the planned surgical procedure, such as by providing risk assessments, suggested courses of action and the like.

Control system 1108 can comprise a controller as described herein, such as, a robotic controller or computing system 140 of FIG. 1. Memory 1122 can comprise a computer readable storage medium having information related to surgical procedure planning for one or more patients. Memory 1122 can comprise databases of bone models, bone density information, prosthesis engineering parameters (e.g., size, weight, dimensions, material properties), numerical modeling equations, kinematic data, prosthesis usage data (e.g., force data for particular activities) and others.

In examples, planning system 1102, surgical system 1104, display device 1106 and control system 1108 can be configured to communicate and exchange data and information with each other. As such, data input into control system 1108 via user interface 1118 (e.g., resection planes, cut depth, prosthesis placement information) can be used by planning system 1102 to operate simulation engine 1110 and simulate surgical outcomes and by artificial intelligence engine 1112 to update simulation engine 1110. Data generated by planning system 1102 can be shared with tracking system 1114 and robot system 1116 to implement a planned surgical procedure.

As mentioned, planning system 1102 can access memory 1122 to access information (e.g., electronic data encoded in a non-transitory computer storage medium) relating to look-up tables for bone density information of bone density markers, anatomic bone density information for healthy and diseased bone such as can be aggregated from bone information of sample populations, force and impact information for joints in performing various activities, size and weight information for various prosthetic implants and devices, and biographic information for patients, as well as numerical modeling algorithms and equations, artificial intelligence and machine learning engines as described herein. Thus, control system 1108 can compute bone density of a specific patient, such as by consulting an imaging of the specific patient, calculate resection planes and fixation feature locations within the patient-specific bone model for a selected prosthesis, determine impact threshold for bone at the resection planes or fixation feature locations, calculate force information for particular activities of the specific patient, provide risk assessment feedback (e.g., potential for bone damage and prosthesis loosening) for the fixation of the selected prosthesis, and provide a recommendation for selecting an implant scenario or selecting a different implant scenario.

FIG. 12 illustrates a block diagram of an example machine 1700 upon which any one or more of the techniques discussed herein may be performed in accordance with some embodiments. For example, machine 1700 can comprise computing system 140 of FIG. 1. Machine 1700 can comprise an example of a controller for planning system 1102 (FIG. 11). As such instructions 1724 can be executed by processor 1702 to generate and bone density information, implant scenarios for various prostheses, and risk assessments of each implant scenario.

Machine 1700 can operate as a standalone device or may be connected (e.g., networked) to other machines. In a networked deployment, machine 1700 may operate in the capacity of a server machine, a client machine, or both in server-client network environments. In an example, machine 1700 can act as a peer machine in peer-to-peer (P2P) (or other distributed) network environment. Machine 1700 may be a personal computer (PC), a tablet PC, a set-top box (STB), a personal digital assistant (PDA), a mobile telephone, a web appliance, a network router, switch or bridge, or any machine capable of executing instructions (sequential or otherwise) that specify actions to be taken by that machine. Further, while only a single machine is illustrated, the term "machine" shall also be taken to include any collection of machines that individually or jointly execute a set (or multiple sets) of instructions to perform any one or more of the methodologies discussed herein, such as cloud computing, software as a service (SaaS), other computer cluster configurations.

Machine 1700 can comprise a computer system and can include hardware processor 1702 (e.g., a central processing unit (CPU), a graphics processing unit (GPU), a hardware processor core, or any combination thereof), main memory 1704 and static memory 1706, some or all of which may communicate with each other via interlink 1708, which can comprise a bus. Machine 1700 may further include display unit 1710, alphanumeric input device 1712 (e.g., a keyboard), and user interface (UI) navigation device 1714 (e.g., a mouse). In an example, display unit 1710, input device 1712 and UI navigation device 1714 may be a touch screen display. Machine 1700 may additionally include storage device (e.g., drive unit) 1716, signal generation device 1718 (e.g., a speaker), network interface device 1720, and one or more sensors 1721, such as a global positioning system (GPS) sensor, compass, accelerometer, or other sensor. Machine 1700 may include output controller 1728, such as a serial (e.g., Universal Serial Bus (USB), parallel, or other wired or wireless (e.g., infrared (IR), near field communication (NFC), etc.) connection to communicate or control one or more peripheral devices (e.g., a printer, card reader, etc.).

Storage device 1716 may include machine readable medium 1722 on which is stored one or more sets of data structures or instructions 1724 (e.g., software) embodying or utilized by any one or more of the techniques or functions described herein. Instructions 1724 may also reside, completely or at least partially, within main memory 1704, within static memory 1706, or within hardware processor 1702 during execution thereof by machine 1700. In an example, one or any combination of hardware processor 1702, main memory 1704, static memory 1706, or storage device 1716 may constitute machine readable media.

While machine readable medium 1722 is illustrated as a single medium, the term "machine readable medium" may include a single medium or multiple media (e.g., a centralized or distributed database, and/or associated caches and servers) configured to store the one or more instructions 1724. The term "machine readable medium" may include any medium that is capable of storing, encoding, or carrying instructions for execution by machine 1700 and that cause machine 1700 to perform any one or more of the techniques of the present disclosure, or that is capable of storing, encoding or carrying data structures used by or associated with such instructions. Non-limiting machine readable medium examples may include solid-state memories, and optical and magnetic media.

Instructions 1724 may further be transmitted or received over communications network 1726 using a transmission medium via network interface device 1720 utilizing any one of a number of transfer protocols (e.g., frame relay, internet protocol (IP), transmission control protocol (TCP), user datagram protocol (UDP), hypertext transfer protocol (HTTP), etc.). Example communication networks may include a local area network (LAN), a wide area network (WAN), a packet data network (e.g., the Internet), mobile telephone networks (e.g., cellular networks), Plain Old Telephone (POTS) networks, and wireless data networks (e.g., Institute of Electrical and Electronics Engineers (IEEE) 802.11 family of standards known as Wi-Fi^{®}, IEEE 802.16 family of standards known as WiMax^{®}), IEEE 802.15.4 family of standards, peer-to-peer (P2P) networks, among others. In an example, network interface device 1720 may include one or more physical jacks (e.g., Ethernet, coaxial, or phone jacks) or one or more antennas to connect to communications network 1726. In an example, network interface device 1720 may include a plurality of antennas to wirelessly communicate using at least one of single-input multiple-output (SIMO), multiple-input multiple-output (MIMO), or multiple-input single-output (MISO) techniques. The term "transmission medium" shall be taken to include any intangible medium that is capable of storing, encoding or carrying instructions for execution by machine 1700, and includes digital or analog communications signals or other intangible medium to facilitate communication of such software.

Bone density databases and prosthetic implant device databases can be stored in main memory 1704 and accessed by processor 1702. Processor 1702 can also receive input (such as at input device 1712) relating to patient-specific bone models including patient-specific bone density information, which can be stored in main memory 1704. Machine 1700 can execute numerical model simulations of prosthesis fixation and can execute AI/ML engines to evaluate fixation of prostheses in bone. Machine 1700 can provide visual indicia of the risks associated with various implant scenarios on display unit 1710 (e.g., interface devices 145). Machine 1700 can be configured to display friction, force, stress and strain information at display unit 1710 to provide real-time feedback to a user when planning the fit of a prosthesis in comparison to predetermined acceptable thresholds for friction, force, stress and strain.

The systems, devices and methods discussed in the present application can be useful in performing prosthetic component procedures to provide better fit and comfort for the patient as well as reducing the potential for unintentionally causing damage to bones into which the prosthetic component is implanted. Bone density information can be used to identify weak or unhealthy bone to plan removal of unhealthy bone and plan implanting of the prosthetic component at healthy bone. Bone density information can additionally be used to accommodate unhealthy bone when it is not possible or acceptable to completely remove or work around unhealthy bone. For example, if it is determined that unhealthy bone should be left in place, then prosthetic devices can be designed and used to improve interfacing with weakened bone and the implantation process can be adapted to minimize trauma to the diseased bone.

In various aspects, the present disclosure can help a surgeon and artificial intelligence or machine learning engines identify weak bone and healthy bone patterns so that the placement of prosthetic devices into bone matter can be selected to improve fixation conditions and improve the long-term post operative outcome.

## Claims

1. A surgical system (1100, 1700) for determining fixation of a prosthesis in bone matter based on bone anatomy and density information, the system comprising: a video display unit (1106, 1710) a control system (1108) having a processor (1120, 1702) and a memory device (1122, 1704, 1716), wherein the memory device has instructions (1724) stored therein configured to cause the processor to perform operations comprising:
generating a three-dimensional model of a bone of a patient for output in the video display unit;
adding three-dimensional bone density information for the bone matter to the three-dimensional model;
simulating a preparation of the bone to produce a bone surface on the three-dimensional model;
simulating placement of the prosthesis in the bone surface;
estimating a fixation strength of the prosthesis to the bone matter using the bone density information at the prosthesis; **characterised in that** the operations further comprise
outputting indicia on the video display unit that indicates risk factors for displacement of the prosthesis from the bone matter.

2. The surgical system of claim 1, wherein generating the three-dimensional model of the bone of the patient comprises aggregating a plurality of three-dimensional scans of the bone; or
wherein generating the three-dimensional model of the bone of the patient comprises 2D to 3D generation using a plurality of two-dimensional x-ray images.

3. The surgical system of claim 1, wherein generating the three-dimensional model of the bone of the patient comprises generating a finite element model;
optionally wherein adding three-dimensional bone density information to the three-dimensional model comprises adding a number representing the grey value of each pixel of the finite element model.

4. The surgical system of any one of claims 1 - 3, wherein simulating placement of a prosthesis in the bone surface comprises:
displaying an outline of the prosthesis in the bone; and
displaying an indication of bone density level at an intersection of the prosthesis with the outline;
optionally the operations further comprising: simulating physical activity of a joint including the bone and the prosthesis; and simulating application of force on the prosthesis due to performance of the physical activity;
further optionally wherein simulating physical activity of a joint including the bone and the prosthesis comprises scaling force data according to patient-specific body information of the patient;
still further optionally wherein the force data is selected from a publicly available database of physical activities or a musculoskeletal model.

5. The surgical system of any one of claims 1 - 4, wherein outputting indicia on the video display unit indicating risk factors for displacement of the prosthesis from the bone surface comprises:
displaying bone density indicators around the prosthesis in a heat map view in the three-dimensional model.

6. The surgical system of any one of claims 1 - 4, wherein
outputting indicia on the video display unit indicating risk factors for displacement of the prosthesis from the bone surface comprises:
displaying a matrix of risk factors and implant conditions including an assessment of
each risk factor at each implant condition;
optionally wherein the assessment comprises an indication of whether or not a threshold for each risk factor has been met.
optionally wherein the risk factors comprise one or more of loosening of the prosthesis, stress shielding of the bone around the prosthesis, dissociation of a component of the prosthesis from another component, bone fracture, implant fracture, bone removal, and cortex perforation.

7. The surgical system of any one of claims 1 - 6, the operations further comprising:
changing an implant parameter;
updating a simulated placement of the prosthesis;
updating a risk factor estimate for the prosthesis; and
updating the indicia of risk factors based on changing of the implant parameter;
optionally further comprising updating the indicia of risk factors in real-time in an operating room;

8. The surgical system of claim 7, optionally wherein changing the implant parameter comprises changing the preparation of the bone surface or changing placement of the prosthesis in the bone surface.

9. The surgical system of claim 8, wherein changing the implant parameter comprises changing a type of the prosthesis.

10. The surgical system of any one of claims 1 - 9, the operations further comprising determining the indicia of risk factors comprises running an artificial intelligence engine to identify weakness in the three-dimensional bone anatomy and density information for the prosthesis;
optionally the operations further comprising updating the artificial intelligence engine with the identified weakness in the three-dimensional bone anatomy and density information;
optionally the operations further comprising running the artificial intelligence engine to estimate the risk factor without simulating the resection and the placement of the prosthesis.

11. A method of generating an electronic surgical plan using a patient-specific bone density model, the method comprising:
obtaining imaging of a bone of a patient including bone anatomy and bone density information of bone matter of the bone;
generating a three-dimensional bone model of the bone of the patient from the imaging;
determining bone density levels of the bone matter at three-dimensional locations in the three-dimensional bone model from bone density information of the imaging;
plotting an interface for a prosthesis on the three-dimensional bone model;
estimating fixation of the prosthesis to the bone at the interface due to engagement with the bone matter; and
saving a digital version of the electronic surgical plan in a computer-readable storage medium, the electronic surgical plan including the prosthesis, the three-dimensional locations of the bone density levels, the interface and estimates of the fixation; **characterised in that** the method further comprises:
comparing fixation levels of the prosthesis to the bone with threshold fixation levels to determine a probability of loosening of the prosthesis from bone; and
displaying visual indicators of the threshold fixation levels on a video output unit.

12. The method of claim 11, wherein estimating fixation of the prosthesis to the bone at the interface due to engagement with the bone matter comprises comparing the three-dimensional location of the bone density to three-dimensional locations of bone density levels of bones of a patient population to determine if the three-dimensional location of the bone density has a greater or lesser amount of bone density than the patient population; or
wherein estimating fixation of the prosthesis to the bone at the interface due to engagement with the bone matter comprises solving equations of a finite element model defining bone density information for the three-dimensional bone model.

13. The method of claim 11, wherein the threshold fixation levels are determined for forces applied to the bone during performance of a specific activities.

14. The method of any one of claims 11 to 13, further comprising:
displaying the three-dimensional bone model on the video output unit;
adjusting the interface for the prosthesis on the three-dimensional bone model in the video output unit or changing a fixation feature of the prosthesis that engages the bone matter;
updating the fixation of the prosthesis to the bone at the adjusted interface; and
updating the displayed visual indicators in real-time.

15. The method of any one of claims 11 - 14, wherein the displayed visual indicators comprise fixation levels between the prosthesis and the bone matter at the interface rendered as a heat map along the interface; and/or
wherein the displayed visual indicators comprise a matrix of risk factors and surgical plan parameters; and/or
further comprising providing a recommendation for selecting the prosthesis or the interface based on comparing the fixation levels of the prosthesis to the bone with the threshold fixation levels; and/or
further comprising updating a database of surgical plans for a patient population with the digital version of the electronic surgical plan to train a machine learning algorithm.

## Patentansprüche

1. Chirurgisches System (1100, 1700) zum Bestimmen einer Fixierung einer Prothese in Knochensubstanz basierend auf Informationen über Knochenanatomie und -dichte, das System umfassend:
eine Videoanzeigeeinheit (1106, 1710), ein Steuersystem (1108), das einen Prozessor (1120, 1702) und eine Speichervorrichtung (1122, 1704, 1716) aufweist, wobei die Speichervorrichtung darin gespeicherte Anweisungen (1724) aufweist, die konfiguriert sind, um den Prozessor zu veranlassen, Vorgänge durchzuführen, umfassend:
Erzeugen eines dreidimensionalen Modells eines Knochens eines Patienten zum Ausgeben in der Videoanzeigeeinheit;
Hinzufügen von dreidimensionalen Knochendichteinformationen für die Knochensubstanz zu dem dreidimensionalen Modell;
Simulieren einer Vorbereitung des Knochens, um eine Knochenoberfläche auf dem dreidimensionalen Modell zu erzeugen;
Simulieren eines Platzierens der Prothese in der Knochenoberfläche;
Schätzen einer Fixierungsstärke der Prothese an der Knochensubstanz unter Verwendung der Knochendichteinformationen an der Prothese;
**dadurch gekennzeichnet, dass** die Vorgänge ferner ein Ausgeben von Hinweisen auf der Videoanzeigeeinheit umfassen, die Risikofaktoren für eine Verlagerung der Prothese von der Knochensubstanz angeben.

2. Chirurgisches System nach Anspruch 1, wobei ein Erzeugen des dreidimensionalen Modells des Knochens des Patienten ein Aggregieren einer Vielzahl von dreidimensionalen Scans des Knochens umfasst; oder
wobei ein Erzeugen des dreidimensionalen Modells des Knochens des Patienten eine 2D-zu-3D-Erzeugung unter Verwendung einer Vielzahl von zweidimensionalen Röntgenbildern umfasst.

3. Chirurgisches System nach Anspruch 1, wobei ein Erzeugen des dreidimensionalen Modells des Knochens des Patienten ein Erzeugen eines Finite-Elemente-Modells umfasst; optional wobei ein Hinzufügen dreidimensionaler Knochendichteinformationen zu dem dreidimensionalen Modell ein Hinzufügen einer Zahl umfasst, die den Grauwert von jedem Pixel des Finite-Elemente-Modells darstellt.

4. Chirurgisches System nach einem der Ansprüche 1-3, wobei ein Simulieren eines Platzierens einer Prothese in der Knochenoberfläche Folgendes umfasst:
Anzeigen eine Umrisses der Prothese in dem Knochen; und
Anzeigen einer Angabe von Knochendichtegrad an einer Schnittstelle der Prothese mit dem Umriss;
optional die Vorgänge ferner umfassend: Simulieren einer körperlichen Aktivität eines Gelenks,
das den Knochen und die Prothese beinhaltet; und Simulieren einer Anwendung von Kraft auf die Prothese aufgrund der Durchführung der körperlichen Aktivität;
ferner optional wobei ein Simulieren körperlicher Aktivität eines Gelenks, das den Knochen und die Prothese beinhaltet, ein Skalieren von Kraftdaten gemäß patientenspezifischer Körperinformationen des Patienten umfasst;
weiter ferner optional ferner wobei die Kraftdaten ausgewählt sind aus einer öffentlich zugänglichen Datenbank körperlicher Aktivitäten oder einem Muskel-Skelett-Modell.

5. Chirurgisches System nach einem der Ansprüche 1-4, wobei ein Ausgeben von Hinweisen auf der Videoanzeigeeinheit, die Risikofaktoren für eine Verlagerung der Prothese von der Knochenoberfläche angeben, Folgendes umfasst:
Anzeigen von Knochendichteindikatoren rund um die Prothese in einer Wärmekartenansicht in dem dreidimensionalen Modell.

6. Chirurgisches System nach einem der Ansprüche 1-4, wobei
ein Ausgeben von Hinweisen auf der Videoanzeigeeinheit, die Risikofaktoren für eine Verlagerung der Prothese von der Knochenoberfläche angeben, Folgendes umfasst:
Anzeige einer Matrix von Risikofaktoren und Implantatbedingungen einschließlich einer Bewertung von jedem Risikofaktors bei jeder Implantatbedingung;
optional wobei ein Bewerten eine Angabe umfasst, ob ein Schwellenwert für jeden Risikofaktor erreicht wurde
optional wobei die Risikofaktoren eines oder mehrere von Lockerung der Prothese, Stressabschirmung des Knochens um die Prothese herum, Ablösung einer Komponente der Prothese von einer anderen Komponente, Knochenbruch, Implantatbruch, Knochenentfernung und Kortexperforation umfassen.

7. Chirurgisches System nach einem der Ansprüche 1-6, die Vorgänge ferner umfassend:
Ändern eines Implantatparameters;
Aktualisieren einer simulierten Platzierung der Prothese;
Aktualisieren einer Risikofaktorschätzung für die Prothese; und
Aktualisieren der Hinweise von Risikofaktoren basierend auf einem Ändern des Implantatparameters;
optional ferner umfassend ein Aktualisieren der Hinweise von Risikofaktoren in Echtzeit in einem Operationssaal.

8. Chirurgisches System nach Anspruch 7, optional wobei ein Ändern des Implantatparameters ein Ändern der Vorbereitung der Knochenoberfläche oder ein Ändern eines Platzierens der Prothese in der Knochenoberfläche umfasst.

9. Chirurgisches System nach Anspruch 8, wobei ein Ändern des Implantatparameters ein Ändern eines Typs der Prothese umfasst.

10. Chirurgisches System nach einem der Ansprüche 1-9, die Vorgänge ferner umfassend ein Bestimmen der Hinweise von Risikofaktoren ein Ausführen einer Maschine künstlicher Intelligenz umfasst, um Schwachstellen in der dreidimensionalen Knochenanatomie und den Dichteinformationen für die Prothese zu identifizieren;
optional die Vorgänge ferner umfassend ein Aktualisieren der Maschine künstlicher Intelligenz mit den identifizierten Schwachstellen in der dreidimensionalen Knochenanatomie und den Dichteinformationen;
optional die Vorgänge ferner umfassend ein Ausführen der Maschine künstlicher Intelligenz, um den Risikofaktor zu schätzen, ohne die Resektion und die Platzierung der Prothese zu simulieren.

11. Verfahren zum Erzeugen eines elektronischen Operationsplans unter Verwendung eines patientenspezifischen Knochendichtemodells, das Verfahren umfassend:
Erlangen von Bildgebung eines Knochens eines Patienten, einschließlich Knochenanatomie- und Knochendichteinformationen von Knochensubstanz des Knochens;
Erzeugen eines dreidimensionalen Knochenmodells des Knochens des Patienten anhand der Bildgebung; Bestimmen von Knochendichtegraden der Knochensubstanz an dreidimensionalen Stellen in dem dreidimensionalen Knochenmodell aus Knochendichteinformationen anhand der Bildgebung;
Aufzeichnen einer Grenzfläche für eine Prothese auf dem dreidimensionalen Knochenmodell;
Schätzen einer Fixierung der Prothese an dem Knochen an der Grenzfläche aufgrund eines Eingriffs mit der Knochensubstanz; und
Speichern einer digitalen Version des elektronischen Operationsplans in einem computerlesbaren Speichermedium, wobei der elektronische Operationsplan die Prothese, die dreidimensionalen Stellen der Knochendichtengrade, die Grenzfläche und Schätzungen der Fixierung beinhaltet;
**dadurch gekennzeichnet, dass** das Verfahren ferner Folgendes umfasst:
Vergleichen von Fixierungsgraden der Prothese an dem Knochen mit den Fixierungsgrad-Schwellenwerten, um eine Wahrscheinlichkeit einer Lockerung der Prothese von dem Knochen zu bestimmen; und
Anzeigen visueller Indikatoren der Fixierungsgrad-Schwellenwerte auf einer Videoausgabeeinheit.

12. Verfahren nach Anspruch 11, wobei ein Schätzen einer Fixierung der Prothese an dem Knochen an der Grenzfläche aufgrund eines Eingriffs mit der Knochensubstanz ein Vergleichen der dreidimensionalen Stelle der Knochendichte mit dreidimensionalen Stellen von Knochendichtegraden von Knochen einer Patientenpopulation umfasst, um zu bestimmen, ob die dreidimensionale Stelle der Knochendichte einen größeren oder geringeren Betrag an Knochendichte als die Patientenpopulation aufweist; oder
wobei ein Schätzen einer Fixierung der Prothese an dem Knochen an der Grenzfläche aufgrund eines Eingriffs mit der Knochensubstanz ein Lösen von Gleichungen eines Finite-Elemente-Modells umfasst, das Knochendichteinformationen für das dreidimensionale Knochenmodell definiert.

13. Verfahren nach Anspruch 11, wobei die Fixierungsgrad-Schwellenwerte für Kräfte bestimmt werden, die während einer Ausübung einer spezifischen Tätigkeit auf den Knochen einwirken.

14. Verfahren nach einem der Ansprüche 11 bis 13, ferner umfassend:
Anzeigen des dreidimensionalen Knochenmodells auf der Videoausgabeeinheit;
Anpassung der Grenzfläche für die Prothese an dem dreidimensionalen Knochenmodell in der Videoausgabeeinheit oder Ändern eines Fixierungsmerkmals der Prothese, das das Knochenmaterial eingreift;
Aktualisieren der Fixierung der Prothese an dem Knochen an der angepassten Grenzfläche; und
Aktualisieren der angezeigten visuellen Indikatoren in Echtzeit.

15. Verfahren nach einem der Ansprüche 11-14, wobei die angezeigten visuellen Indikatoren Fixierungsgrade zwischen der Prothese und der Knochensubstanz an der Grenzfläche umfassen, die als Wärmekarte entlang der Grenzfläche aufgezeigt werden; und/oder wobei die angezeigten visuellen Indikatoren eine Matrix von Risikofaktoren und Operationsplanparametern umfassen; und/oder
ferner umfassend ein Bereitstellen einer Empfehlung zum Auswählen der Prothese oder der Grenzfläche basierend auf einem Vergleichen der Fixierungsgrade der Prothese an dem Knochen mit den Fixierungsgrad-Schwellenwerten; und/oder
ferner umfassend ein Aktualisieren einer Datenbank von Operationsplänen für eine Patientenpopulation mit der digitalen Version des elektronischen Operationsplans, um einen maschinellen Lernalgorithmus zu trainieren.

## Revendications

1. Système chirurgical (1100, 1700) permettant de déterminer la fixation d'une prothèse dans la matière osseuse sur la base des informations sur la densité et l'anatomie osseuses, le système comprenant :
une unité d'affichage vidéo (1106, 1710),
un système de commande (1108) comportant un processeur (1120, 1702) et un dispositif de mémoire (1122, 1704, 1716), ledit dispositif de mémoire stockant des instructions (1724) configurées pour amener le processeur à effectuer des opérations comprenant :
la génération d'un modèle tridimensionnel d'un os d'un patient en vue de son émission dans l'unité d'affichage vidéo ;
l'ajout d'informations sur la densité osseuse tridimensionnelle pour la matière osseuse au modèle tridimensionnel ;
la simulation d'une préparation de l'os pour produire une surface osseuse sur le modèle tridimensionnel ;
la simulation du placement de la prothèse dans la surface osseuse ;
l'estimation d'une force de fixation de la prothèse à la matière osseuse à l'aide des informations sur la densité osseuse au niveau de la prothèse ;
**caractérisé en ce que** les opérations comprennent en outre l'émission d'indices sur l'unité d'affichage vidéo qui indiquent des facteurs de risque de déplacement de la prothèse par rapport à la matière osseuse.

2. Système chirurgical de la revendication 1, ladite génération du modèle tridimensionnel de l'os du patient comprenant l'agrégation d'une pluralité de balayages tridimensionnels de l'os ; ou
ladite génération du modèle tridimensionnel de l'os du patient comprenant la génération 2D à 3D à l'aide d'une pluralité d'images radiographiques bidimensionnelles.

3. Système chirurgical de la revendication 1, ladite génération du modèle tridimensionnel de l'os du patient comprenant la génération d'un modèle d'éléments finis ;
éventuellement ledit ajout d'informations sur la densité osseuse tridimensionnelle au modèle tridimensionnel comprenant l'ajout d'un nombre représentant la valeur de gris de chaque pixel du modèle d'éléments finis.

4. Système chirurgical de l'une quelconque des revendications 1 à 3, ladite simulation du placement d'une prothèse dans la surface osseuse comprenant :
l'affichage du contour de la prothèse dans l'os ; et
l'affichage d'une indication du niveau de densité osseuse au niveau d'une intersection de la prothèse avec le contour ;
éventuellement, lesdites opérations comprenant en outre : la simulation de l'activité physique d'une articulation comprenant l'os et la prothèse ; et la simulation de l'application d'une force sur la prothèse en raison de la réalisation de l'activité physique ;
en outre, éventuellement, ladite simulation de l'activité physique d'une articulation comprenant l'os et la prothèse comprenant des données de force de mise à l'échelle en fonction des informations corporelles spécifiques au patient du patient ;
en outre, éventuellement encore, lesdites données de force étant sélectionnées à partir d'une base de données disponible au public d'activités physiques ou d'un modèle musculo-squelettique.

5. Système chirurgical de l'une quelconque des revendications 1 à 4, ladite émission d'indices sur l'unité d'affichage vidéo indiquant des facteurs de risque de déplacement de la prothèse par rapport à la surface osseuse comprenant :
l'affichage des indicateurs de densité osseuse autour de la prothèse dans une vue de carte thermique dans le modèle tridimensionnel.

6. Système chirurgical de l'une quelconque des revendications 1 à 4,
ladite émission d'indices sur l'unité d'affichage vidéo indiquant des facteurs de risque de déplacement de la prothèse par rapport à la surface osseuse comprenant :
l'affichage d'une matrice de facteurs de risque et des conditions d'implant y compris une évaluation de chaque facteur de risque à chaque condition d'implant ;
éventuellement ladite évaluation comprenant une indication indiquant si un seuil pour chaque facteur de risque a été atteint ou non,
éventuellement lesdits facteurs de risque comprenant un ou plusieurs facteurs parmi le desserrage de la prothèse, le bouclier anti-contraintes de l'os autour de la prothèse, la dissociation d'un composant de la prothèse par rapport à un autre composant, la fracture de l'os, la fracture de l'implant, l'ablation de l'os et la perforation du cortex.

7. Système chirurgical de l'une quelconque des revendications 1 à 6, lesdites opérations comprenant en outre :
la modification d'un paramètre d'implant ;
la mise à jour d'un placement simulé de la prothèse ;
la mise à jour d'une estimation du facteur de risque pour la prothèse ; et
la mise à jour des indices de facteurs de risque en fonction de la modification du paramètre de l'implant ;
comprenant en outre éventuellement la mise à jour des indices de facteurs de risque en temps réel dans une salle d'opération.

8. Système chirurgical de la revendication 7, éventuellement ladite modification du paramètre d'implant comprenant la modification de la préparation de la surface osseuse ou la modification du placement de la prothèse dans la surface osseuse.

9. Système chirurgical de la revendication 8, ladite modification du paramètre de l'implant comprenant la modification d'un type de la prothèse.

10. Système chirurgical de l'une quelconque des revendications 1 à 9, lesdites opérations comprenant en outre la détermination des indices de facteurs de risque comprenant l'exécution d'un moteur d'intelligence artificielle pour identifier une faiblesse dans les informations sur la densité et l'anatomie osseuse tridimensionnelle pour la prothèse ;
éventuellement, lesdites opérations comprenant en outre la mise à jour du moteur d'intelligence artificielle avec la faiblesse identifiée dans les informations sur la densité et l'anatomie osseuse tridimensionnelle ;
éventuellement, lesdites opérations comprenant en outre l'exécution du moteur d'intelligence artificielle pour estimer le facteur de risque sans simuler la résection et le placement de la prothèse.

11. Procédé de génération d'un plan chirurgical électronique à l'aide d'un modèle de densité osseuse spécifique au patient, le procédé comprenant :
l'obtention d'une imagerie d'un os d'un patient comprenant des informations sur l'anatomie osseuse et la densité osseuse de la matière osseuse de l'os ;
la génération d'un modèle osseux tridimensionnel de l'os du patient à partir de l'imagerie ;
la détermination des niveaux de densité osseuse de la matière osseuse à des emplacements tridimensionnels dans le modèle osseux tridimensionnel à partir des informations sur la densité osseuse de l'imagerie ;
le traçage d'une interface pour une prothèse sur le modèle osseux tridimensionnel ;
l'estimation de la fixation de la prothèse à l'os au niveau de l'interface en raison de l'engagement avec la matière osseuse ; et
l'enregistrement d'une version numérique du plan chirurgical électronique dans un support de stockage lisible par ordinateur, le plan chirurgical électronique comprenant la prothèse, les emplacements tridimensionnels des niveaux de densité osseuse, l'interface et les estimations de la fixation ;
**caractérisé en ce que** le procédé comprend en outre :
la comparaison des niveaux de fixation de la prothèse à l'os avec des niveaux de fixation de seuil pour déterminer une probabilité de desserrage de la prothèse par rapport à l'os ; et
l'affichage d'indicateurs visuels des niveaux de fixation de seuil sur une unité de sortie vidéo.

12. Procédé de la revendication 11, ladite estimation de la fixation de la prothèse à l'os au niveau de l'interface en raison de l'engagement avec la matière osseuse comprenant la comparaison de l'emplacement tridimensionnel de la densité osseuse à des emplacements tridimensionnels des niveaux de densité osseuse des os d'une population de patients pour déterminer si l'emplacement tridimensionnel de la densité osseuse présente une quantité plus ou moins grande de densité osseuse que la population de patients ; ou
ladite estimation de la fixation de la prothèse à l'os au niveau de l'interface en raison de l'engagement avec la matière osseuse comprenant la résolution d'équations d'un modèle d'éléments finis définissant des informations sur la densité osseuse pour le modèle osseux tridimensionnel.

13. Procédé de la revendication 11, lesdits niveaux de fixation de seuil étant déterminés pour les forces appliquées sur l'os lors de l'exécution d'une activité spécifique.

14. Procédé de l'une quelconque des revendications 11 à 13, comprenant en outre :
l'affichage du modèle osseux tridimensionnel sur l'unité de sortie vidéo ;
l'ajustement de l'interface pour la prothèse sur le modèle osseux tridimensionnel dans l'unité de sortie vidéo ou la modification d'une caractéristique de fixation de la prothèse qui engage la matière osseuse ;
la mise à jour de la fixation de la prothèse à l'os au niveau de l'interface ajustée ; et
la mise à jour des indicateurs visuels affichés en temps réel.

15. Procédé de l'une quelconque des revendications 11 à 14, lesdits indicateurs visuels affichés comprenant des niveaux de fixation entre la prothèse et la matière osseuse au niveau de l'interface rendus sous la forme d'une carte thermique le long de l'interface ; et/ou
lesdits indicateurs visuels affichés comprenant une matrice de facteurs de risque et des paramètres de plan chirurgical ; et/ou
comprenant en outre la fourniture d'une recommandation pour la sélection de la prothèse ou de l'interface sur la base de la comparaison des niveaux de fixation de la prothèse à l'os avec les niveaux de fixation de seuil ; et/ou
comprenant en outre la mise à jour d'une base de données de plans chirurgicaux pour une population de patients avec la version numérique du plan chirurgical électronique pour former un algorithme d'apprentissage automatique.
